(19) Europäisches Patentamt
European Patent Office
Office européen des brevets

(11) Publication number: **0 067 565**
**A1**

# (12) EUROPEAN PATENT APPLICATION

(21) Application number: 82302660.4

(22) Date of filing: 25.05.82

(51) Int. Cl.³: **C 07 D 471/04**
C 07 D 455/02, A 61 K 31/435
//(C07D471/04, 221/00, 209/00)

(30) Priority: 16.06.81 GB 8118483
28.10.81 GB 8132416

(43) Date of publication of application:
22.12.82 Bulletin 82/51

(84) Designated Contracting States:
BE CH DE FR GB IT LI NL SE

(71) Applicant: BEECHAM GROUP PLC
Beecham House Great West Road
Brentford Middlesex(GB)

(72) Inventor: Hadley, Michael Stewart
9 Coney Gree
Sawbridgeworth Herts(GB)

(72) Inventor: King, Francis David
67 Cherry Garden Lane
Newport Essex(GB)

(74) Representative: Stott, Michael John et al,
European Patent Attorney Beecham Pharmaceuticals
Great Burgh Yew Tree Bottom Road
Epsom Surrey KT18 5XQ(GB)

## (54) Pharmaceutically active compounds.

(57) Compounds of formula (I)

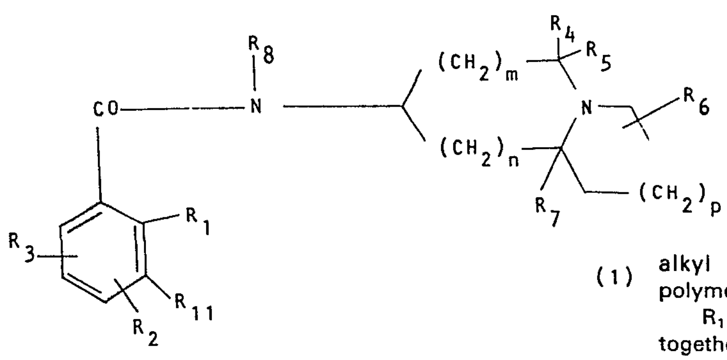

(1)

and pharmaceutically acceptable salts and N-oxides thereof, wherein:

$R_1$ is $C_{1-6}$ alkoxy, $C_{1-6}$ alkylthio or, together with $R_8$ is $C_{1-2}$ alkylene;

one of $R_2$, $R_3$ and $R_{11}$ is hydrogen and the other two together are $C_{1-2}$ alkylenedioxy, or the other two are the same or different and are selected from the class of hydrogen, halogen, $CF_3$, $C_{1-6}$ alkyl, $C_{1-6}$ alkoxy, $C_{1-6}$ alkylthio, $C_{1-7}$ acyl, $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups or optionally N-disubstituted by $C_{4-5}$ polymethylene; or

$R_1$ and $R_{11}$ together are $C_{1-2}$ alkylenedioxy and $R_2$ and $R_3$ together are $C_{1-2}$ alkylenedioxy or $R_2$ and $R_3$ and the same or different and are selected from the previously defined class of substituents;

$R_4$, $R_5$, $R_6$ and $R_7$ are the same or different and are hydrogen, $C_{1-6}$ alkyl, phenyl or phenyl-$C_{1-4}$ alkyl, either of which phenyl moieties may be substituted by $C_{1-6}$ alkyl, $C_{1-6}$ alkoxy, $CF_3$ or halogen, and at least one of $R_4$, $R_5$ and $R_7$ is other than hydrogen;

$R_8$ is hydrogen or $C_{1-4}$ alkyl or as defined with $R_1$;

p is 0 to 3;

m is 0 to 3; and

n is 0 to 3 having pharmacological activity, a process for their preparation and their use.

EP 0 067 565 A1

BICYCLIC BENZAMIDES, A PROCESS FOR THEIR PREPARATION AND THEIR USE

This invention relates to a group of novel compounds, as process for their preparation, their formulation as pharmaceutical compositions, and their use in the therapy of disorders.

West German Offenlegungsschrift No: 2,748,260.6 describes compounds of the formula (A), and pharmaceutically acceptable salts thereof:

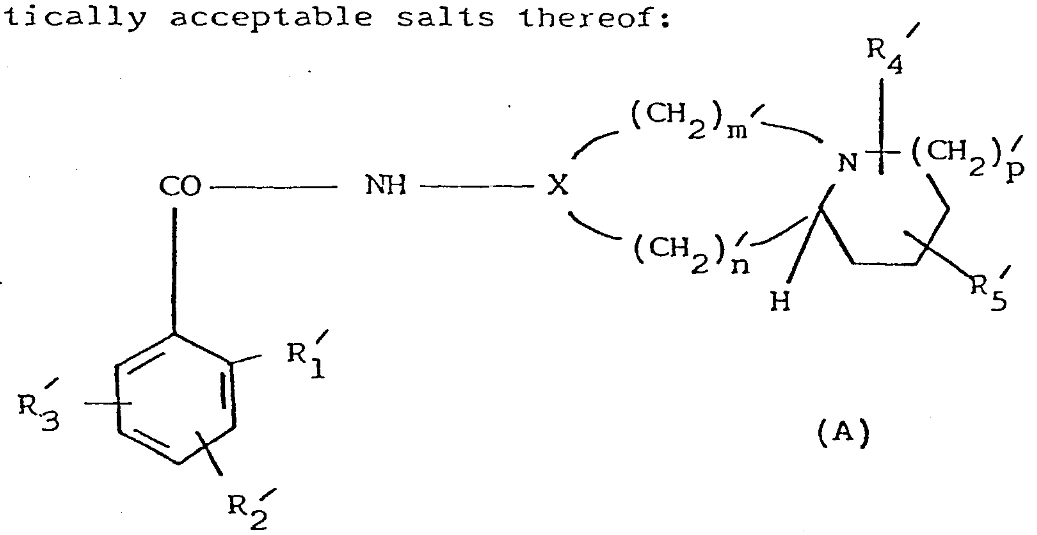

(A)

wherein:

$R_1'$ is a $C_{1-6}$ alkoxy group;

$R_2'$ and $R_3'$ are the same or different and are hydrogen, halogen, $CF_3$, hydroxy, $C_{1-6}$ alkoxy, $C_{2-7}$ acyl, amino, amino substituted by one or two $C_{1-6}$ alkyl groups, $C_{2-7}$ acyl amino, aminocarbonyl or aminosulphone optionally substituted by one or two $C_{1-6}$ alkyl groups, $C_{1-6}$ alkylsulphone or nitro groups;

BAD ORIGINAL

X is either a nitrogen atom, in which case $m'+n'$ is 3 to 5, $m'$ is 2 to 4 and $n'$ is 1 to 3; or X is CH in which case $m'+n'$ is 2 to 5, $m'$ is 1 to 5, and $n'$ is 0 to 4;

... p is 0 to 3; and

$R_4'$ is hydrogen, $C_{1-6}$ alkyl, phenyl or phenyl-$C_{1-6}$ alkyl, either of which phenyl moiety may be substituted by $C_{1-6}$ alkyl, $C_{1-6}$ alkoxy, $CF_3$ or halogen, and $R_5'$ is hydrogen; or $R_4'$ and $R_5'$ are attached to two adjacent carbon atoms and form together with these two carbon atoms a fused benzene ring, which benzene ring may be substituted by $C_{1-6}$ alkyl, $C_{1-6}$ alkoxy, $CF_3$ or halogen; as dopamine antagonists having use in the treatment of disorders of the gastro-intestinal function and/or in the treatment of emesis.

A novel class of compounds has now been discovered which is structurally distinct from the known compounds of the formula (I) but also has useful pharmacological activity, such as dopamine antagonist activity.

Accordingly the present invention provides a compound of the formula (I):

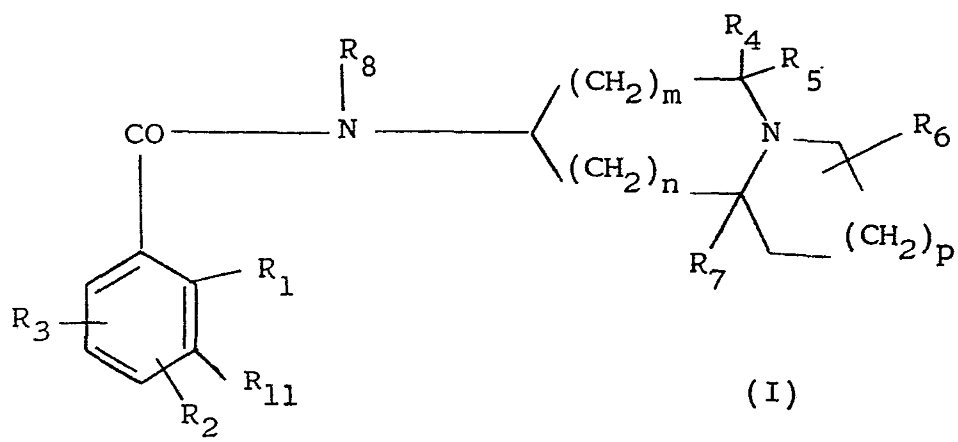

(I)

BAD ORIGINAL

and pharmaceutically acceptable salts and N-oxides thereof, wherein:

$R_1$ is $C_{1-6}$ alkoxy, $C_{1-6}$ alkylthio or, together with $R_8$ is $C_{1-2}$ alkylene;

one of $R_2$, $R_3$ and $R_{11}$ is hydrogen and the other two together are $C_{1-2}$ alkylenedioxy, or the other two are the same or different and are selected from the class of hydrogen, halogen, $CF_3$, $C_{1-6}$ alkyl, $C_{1-6}$ alkoxy, $C_{1-6}$ alkythio, $C_{1-7}$ acyl, $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups or optionally N-disubstituted by $C_{4-5}$ polymethylene; or

$R_1$ and $R_{11}$ together are $C_{1-2}$ alkylenedioxy and $R_2$ and $R_3$ together are $C_{1-2}$ alkylenedioxy or $R_2$ and $R_3$ and the same or different and are selected from the previously defined class of substituents;

$R_4$, $R_5$, $R_6$ and $R_7$ are the same or different and are hydrogen, $C_{1-6}$ alkyl, phenyl or phenyl-$C_{1-4}$ alkyl, either of which phenyl moieties may be substituted by $C_{1-6}$ alkyl, $C_{1-6}$ alkoxy, $CF_3$ or halogen, and at least one of $R_4$, $R_5$ and $R_7$ is other than hydrogen;

$R_8$ is hydrogen or $C_{1-4}$ alkyl or as defined with $R_1$;

p is 0 to 3;

m is 0 to 3; and

n is 0 to 3.

Suitable examples of the group $R_1$ include methoxy, ethoxy <u>n</u>- and <u>iso</u>-propoxy, methylthio, ethylthio and <u>n</u> and <u>iso</u>-propylthio, or together with $R_8$ is methylene. Preferably $R_1$ is a methoxy group.

Suitable examples of $R_2$, $R_3$ and $R_{11}$ include the following atoms and groups: hydrogen; chlorine, bromine; $CF_3$;

formyl, acetyl, propionyl, n- and iso-butyryl; formylamino, acetylamino, propionylamino, n- and iso-butyrylamino; methyl, ethyl and n- and iso-propylsulphone, -sulphinyl or -thia; nitro; methoxy, ethoxy and n- and iso-propoxy; hydroxy; amino, aminocarbonyl and aminosulphonyl and amino, aminocarbonyl, and aminosulphonyl substituted by one or two methyl, ethyl, n- or iso-propyl, cyclopropyl, cyclobutyl, cyclopentyl, cyclohexyl, cycloheptyl, phenyl, or benzyl groups.

When $R_1$ and $R_{11}$ taken together are methylenedioxy or ethylenedioxy, they are most suitably ethylenedioxy.

Particularly suitable $R_2$ $R_3$ and $R_{11}$ groups include hydrogen, halogen and amino; and as "intermediates", acylamino and nitro, which can conveniently be converted to the corresponding amino groups.

It is generally preferred that $R_2$ when $R_{11}$ is hydrogen, is in the 4-position relative to the carbonyl side chain for greater activity in the resultant compound of the formula (I). For the same reason, it is generally preferred that $R_3$ is inthe 5-position relative to the carbonyl side chain.

Particularly preferred $R_2$ groups include 4-amino and 4-acylamino. Most preferably $R_2$ is 4-amino. Particularly preferred $R_3$ groups include 5-halo, such 5-chloro.

In other useful compounds $R_{11}$ is hydrogen and $R_2$ is hydrogen, 4-halo (eg chloro), or amino; and $R_3$ is 5-$C_{1-6}$ alkyl $S(O)_n$ (such as 5-methylsulphonyl, -sulphinyl or -this) or 5-optionally substituted aminosulphonyl.

Suitable examples of $R_4$, $R_5$, $R_6$ and $R_7$ include hydrogen, methyl, ethyl, n- and iso-propyl, phenyl and benzyl. Phenyl groups in $R_4$, $R_5$, $R_6$ and $R_7$ may be substituted by one or more $C_{1-4}$ alkoxy (such as ethoxy), $C_{1-4}$ alkyl (such as methyl), fluoro, chloro or $CF_3$. Often $R_4$, $R_5$, $R_6$ and $R_7$ will be selected from hydrogen, $C_{1-4}$ alkyl or phenyl. Most suitably $R_4$, $R_5$, $R_6$ and $R_7$ are either hydrogen or methyl.

In one distinct sub-class of compounds of the formula (I) $R_7$ is hydrogen; $R_4$ is $C_{1-6}$ alkyl, phenyl or phenyl $C_{1-4}$ alkyl, either of which phenyl moiety may be substituted (as previously defined); and $R_5$ is hydrogen, $C_{1-6}$ alkyl, phenyl or phenyl $C_{1-4}$ alkyl, either of which phenyl moiety may be substituted (as previously defined).

In a second distinct sub-class of compounds of the formula (I) $R_4$ and $R_5$ are both hydrogen, and $R_7$ is $C_{1-6}$ alkyl, phenyl or phenyl $C_{1-4}$ alkyl, either of which phenyl moiety may be substituted (as previously defined).

Suitable examples of $R_8$ include hydrogen, methyl, ethyl and _n_- and _iso_-propyl. More suitably $R_8$ is hydrogen or methyl, preferably hydrogen.

Suitable values for p include 1 and 2, preferably 1.

Suitable values for n include 0, 1 and 2, preferably 1.

Suitable values for m include 0 and 1, preferably 1.

It is generally preferred for higher activity that the bond between the benzamide moiety and the cyclic side chain (ie between the $R_8$ substituted nitrogen atom and the ring carbon atom to which this nitrogen atom is joined) in the compounds of formula (I) equatorial (when used herein in relation to this bond, equatorial is assigned with the bicyclic drawn with a trans ring fusion, and is also referred to as β. Axial is similarly assigned and is also referred to as α).

The pharmaceutically acceptable salts of the compound of the formula (I) include acid addition salts with conventional acids such as hydrochloric, hydrobromic, phosphoric, sulphuric, citric, tartaric, lactic and acetic acid.

The pharmaceutically acceptable salts of the compounds of the formula (I) also include quaternary ammonium salts. Examples of such salts include salts with compounds such as $R_{10}$ - Y wherein $R_{10}$ is $C_{1-6}$ alkyl, phenyl - $C_{1-6}$ alkyl or $C_{5-7}$ cycloalkyl, and Y is an anion of an acid. Suitable examples of $R_{10}$ include methyl, ethyl and n- and iso-propyl; and benzyl and phenyl ethyl. Suitable examples of Y include the halides such as chloride, bromide and iodide.

There is a group of compounds within formula (I) wherein:

$R_1$ is a $C_{1-6}$ alkoxy group;

$R_2$ and $R_3$ are the same or different and are hydrogen, halogen, $CF_3$, $C_{1-7}$ acyl, $C_{1-7}$ acylamino, $C_{1-6}$ alkyl $S(O)_n$ wherein n is O, 1 or 2 nitro, $C_{1-6}$ alkoxy, hydroxy, or amino, aminocarbonyl or aminosulphonyl optionally substituted by one or two $C_{1-6}$ alkyl groups;

or $R_1$ and $R_2$ taken together are methylenedioxy or ethylenedioxy in which case $R_3$ is any one of the groups given for $R_1$ and $R_2$ above;

$R_8$ is hydrogen or $C_{1-4}$ alkyl; and $R_4$, $R_5$, $R_6$, $R_7$, p, m and n are as defined in formula (I)

From the aforesaid it will be seen that suitably the moiety of formula (II):

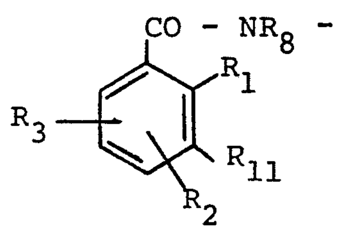

(II)

in a compound of the formula (I) will have the structure (III):

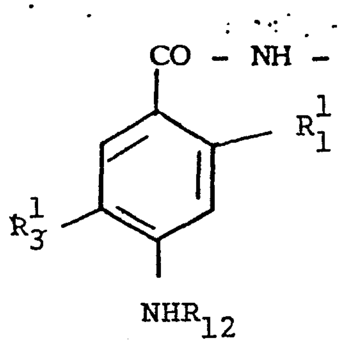

(III)

wherein $R_1^1$ is $C_{1-6}$ alkoxy;

$R_{12}$ is hydrogen or $C_{1-4}$ alkanoyl; and

$R_3^{'}$ is halogen.

Suitable and preferred values for $R_1^1$ are as hereinbefore described under formula (I) for $R_1$ when $C_{1-6}$ alkoxy

Suitable values for $R_{12}$ include acetyl and propionyl; preferably acetyl. $R_3$ is preferably chloro.

From the aforesaid it will also be appreciated that in one suitable sub-group of compounds of formula (I) the moiety of formula (IV):

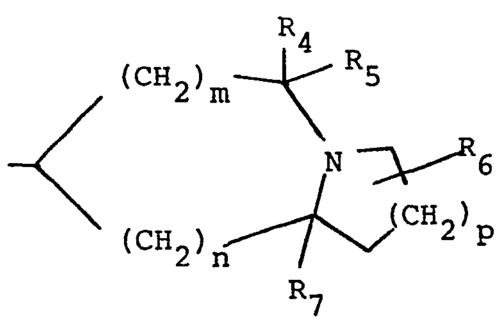

(IV)

is of the formula (V):

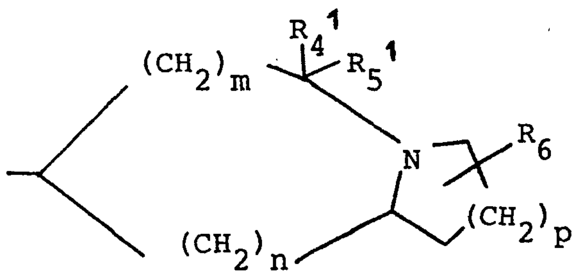

(V)

wherein $R_4^1$ is $C_{1-6}$ alkyl, phenyl or phenyl $C_{1-4}$ alkyl, either of which phenyl moieties may be substituted (as hereinbefore defined); and

$R_5^1$ is hydrogen, $C_{1-6}$ alkyl, phenyl or phenyl $C_{1-4}$ alkyl, either of which phenyl moieties may be substituted (as hereinbefore defined).

$R_6$, m, n and p are as hereinbefore defined.

Suitable and preferred values for $R_4^1$ and $R_5^1$ are as hereinbefore described for $R_4$ and $R_5$.

Preferably one or both of $R_4^1$ and $R_5^1$ are methyl.

Suitable and preferred values for $R_6$, m, n and p are as hereinbefore described in formula (I).

The moiety of formula (V) may favourably be of formula (VI):

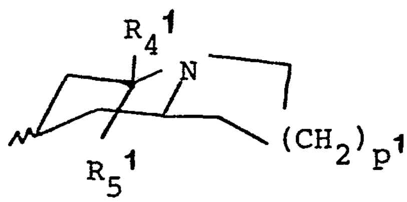

(VI)

wherein $p^1$ is 1 or 2, preferably 1 and $R_4^1$ and $R_5^1$ are as hereinbefore defined

$R_5^1$ is preferably hydrogen.

Thus one favourable sub-group of compounds within formula (I) is of formula (VII):

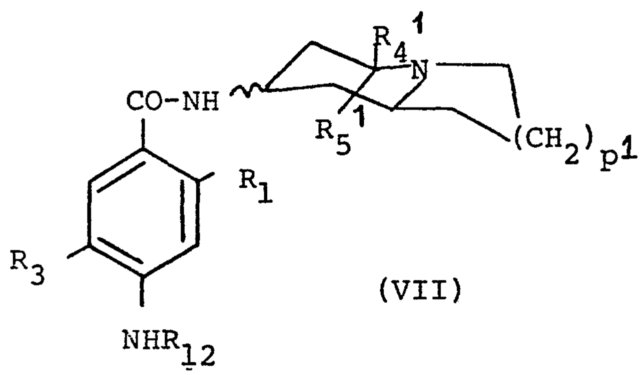

(VII)

In formula (VII), the bond indicated may be axial or equatorial.

In another suitable sub-group of compounds of formula (I) the moiety of formula (IV) is of formula (VIII):

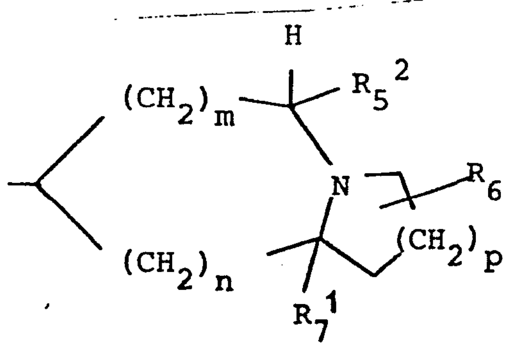

(VIII)

wherein $R_5^2$ is hydrogen, $C_{1-6}$ alkyl, phenyl or phenyl $C_{1-4}$ alkyl, either of which phenyl moieties may be substituted (as hereinbefore defined); and

$R_7^1$ is $C_{1-6}$ alkyl, phenyl or phenyl $C_{1-4}$ alkyl, either of which phenyl moieties may be substituted (as hereinbefore defined).

$R_6$, m, n and p are as hereinbefore defined.

Suitable values for $R_5^2$ and $R_7^1$ are as hereinbefore described for $R_5$ and $R_7$.

Very often $R_5^2$ will be hydrogen.

Preferably $R_5^2$ is hydrogen and $R_7^1$ is methyl.

Suitable and preferred values for $R_6$, m, n and p are as hereinbefore described in formula (I).

The moiety of formula (VII) may favourably be of formula (IX):

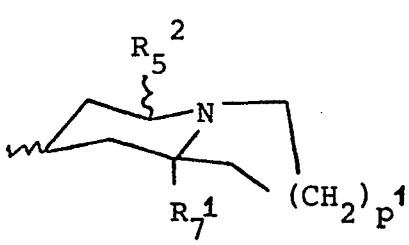

(IX)

wherein $R_5^2$ , $R_7^1$ and $p^1$ are as hereinbefore defined.

$R_5^2$ may suitably be axial or equatorial, and is very often hydrogen.

Thus another favourable sub-group of compounds of formula (I) is of formula (X):

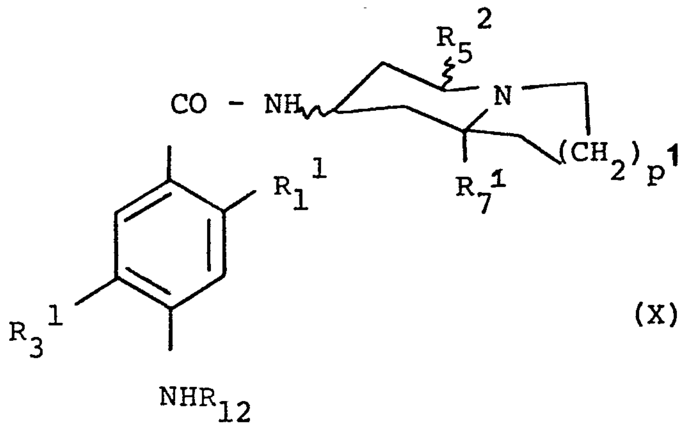

(X)

In formula (X) the bonds indicated may be axial or equatorial.

It will, of course, be realised that the compounds of the formula (I) have chiral centres, and thus are capable of existing in a number of stereoisomeric forms. The invention extends to each of these stereoisomeric forms, and to mixtures thereof (including racemates). The different stereoisomeric forms may be separated one from the other by the usual methods, or any given isomer may be obtained by stereospecific or asymmetric synthesis.

The invention also provides a process for the preparation of a compound of the formula (I), which process comprises reacting a compound of the formula (XI):

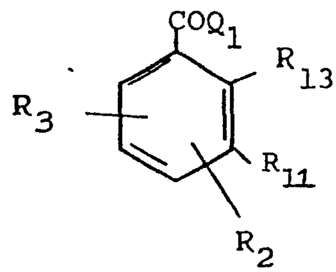

(XI)

with a compound of formula (XII):

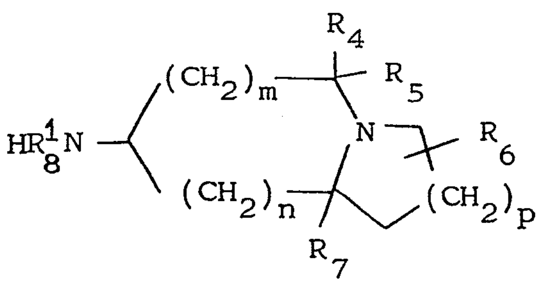

(XII)

wherein $Q_1$ is a leaving group;

$R_{13}$ is $R_1$ as defined or $(CH_2)_qCR_{14}R_{15}$ $Q_2$ wherein q is O or 1, $R_{14}$ and $R_{15}$ are both hydrogen or together form an oxo group and $Q_2$ is a leaving group or $Q_1$ and, $Q_2$ are joined to form a bridging oxygen atom;

$R_8^1$ is hydrogen or $C_{1-4}$ alkyl; and the remaining variable groups are as defined in formula (I); and thereafter when $R_{13}$ is $(CH_2)_qCR_{14}R_{15}$ $Q_2$ and $R_{15}$ form an oxo group, reducing the resulting compound and optionally converting a group $R_2$,$R_3$ or $R_{11}$ in the thus formed compound to another group $R_2$,$R_3$ or $R_{11}$ respectively.

The leaving groups $Q_1$ and $Q_2$ are groups that are readily displaceable by a nucleophile. Examples of such groups are hydroxy, halogen such as chloro and bromo, and for $Q_1$ and $Q_2$ when $R_{14}$ and $R_{15}$ together with the carbon atom to which they are attached are a carbonyl group, acyloxy such as $C_{1-4}$alkanoyloxy, $C_{1-4}$ alkoxycarbonyloxy activated hydrocarbyloxy such as pentachlorophenoxy.

If a leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as -10 to 100$^{o}$C, for example 0 to 80$^{o}$C.

If a leaving group is a halide, then the reaction is preferably carried out at a non-extreme temperature in an inert non-hydroxylic solvent, such as benzene, toluene or diethyl ether. It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate.

If a leaving group is acyloxy, then the reaction is preferably carried in substantially the same manner as if the leaving group were hydroxy. Suitable examples of acyloxy leaving groups include $C_{1-4}$alkanoyloxy, mesyloxy, tosyloxy and triflate.

If a leaving group is $C_{1-4}$ alkoxycarbonyloxy, then the reaction is preferably carried out in an inert solvent, such as methylene chloride, at a non-extreme temperature in the presence of an

BAD ORIGINAL

acid acceptor, such as triethylamine.

If a leaving group is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

Preferably $Q_1$ when $R_{13}$ is $R_1$, is halogen such as chloro.

When $R_{14}$ and $R_{15}$ are both hydrogen, $Q_1$ and $Q_2$ are the same or different and preferably are halogen, mesyloxy, tosyloxy, triflate or hydroxy. $Q_1$ may also be $C_{1-4}$ alkanoyloxy or activated hydrocarbyloxy. Conveniently, $Q_1$ and $Q_2$ are the same, in particular the same halogen.

When $R_{14}$ and $R_{15}$ together from an oxo group, $Q_2$ is preferably an activated hydrocarbyloxy group. Alternatively, $Q_1$ and $Q_2$ together are O, thus forming a derivative of phtalic anhydride (when q=0) or of homophthalic anhydride (when q=1).

Where $R_{14}$ and $R_{15}$ together form an oxo group, the reduction of the carbonyl group in the thus formed compound is preferably carried out, with or without isolation of the compound, by reduction with tin/hydrochloric acid at a non-extreme temperature.

It will be realised that in the compound of the formula (I) the linkage between the $-NR_8-$ moiety and the cyclic side chain may have an α or β orientation with respect to the ring of the bicyclic moiety to which it is attached. A mixture of α and β isomers of the compound of the formula (I) may be synthesised non-stereospecifically and the desired isomer separated conventionally therefrom, e.g. by chromatography; or alternatively the α or β isomer may if desired be synthesised from the corresponding α or β form of the compound of the formula (XII).

Alternatively, a mixture of the α and β isomers of the compound of the formula (XII) may be synthesised non-stereospecifically and the desired isomer separated conventionally therefrom, e.g. by chromatography. However, in this case it is generally more convenient to react the mixture to give a mixture of α and β isomers of the compound of the formula (I) and to separate these if desired as hereinbefore described.

The intermediates of formula (XII) may be prepared by a process which depends on whether the α or β forms is desired, or a mixture thereof.

To prepare α or axial compounds of formula (XII), a compound of formula (XIII) is reduced:

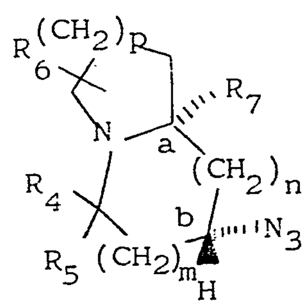

(XIII)

This reduction is conveniently carried out with lithium aluminium hydride under the usual conditions.

Compounds of formula (XIII) may themselves be prepared by reaction of a compound of the formula (XIV):

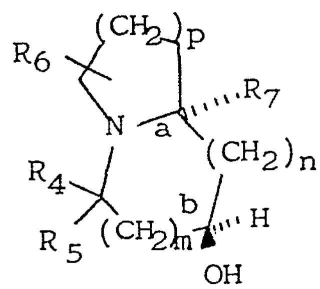

(XIV)

to effect conversion of hydroxy to azido, with inversion of sterochemistry.

This reaction may be carried out in the general manner described in A.K. Bose et. al., Tetrahedron Letters 1977, 23, 1977). For example, the reaction may be effected with triphenylphosphine, diethyl azodicarboxylate and diphenylphosphoryl azide.

Compounds of the formula (XIV) are important intermediates, as from these compounds the β form of compounds of the formula (XIV) and α/β mixtures thereof, may be prepared.

For example, reaction of a compound of formula (XIV) with a base and fluorenone yields a compound of formula (XV):

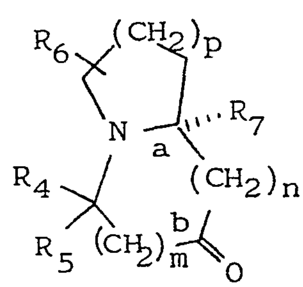

(XV)

The compounds of formula (XV) may be converted to compounds of formula (XVI):

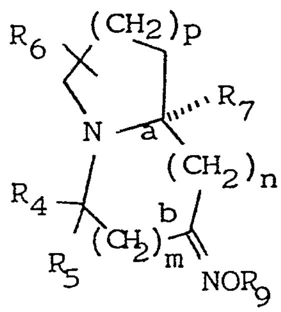

(XVI)

wherein $R_9$ is hydrogen or $C_{1-4}$ alkyl or phenyl $C_{1-4}$ alkyl;

(Suitable values for $R_9$ include methyl and benzyl) with the appropriate hydroxylamine. We have found that this reaction is suitably carried out with pyridine and the appropriate hydroxylamine hydrochloride at reflux temperatures.

Reduction of a compound of formula (XVI) wherein $R_9$ is hydrogen with lithium aluminium hydride gives a mixture of axial (α) and equatorial (β) amines of formula (XII).

In contrast, a dissolving metal reduction of a compound of formula (XVI) is substantially stereospecific and yields mainly the β amines of formula (XII) (any minor proportions of the undesired isomer prepared in this process can be separated in conventional manner). Such reductions may suitably be carried out with sodium and amyl alcohol.

When $R_9$ is $C_{1-4}$ alkyl or phenyl-$C_{1-4}$alkyl, reduction with diisobutyl aluminium hydride yields mainly the α-(axial) amine of formula (XII).

Compounds of the formula (XIV) may be prepared by conventional methods. For example, compounds of formula (XIV) wherein n and m are both 1 may be prepared from a compound of formula (XVII):

 

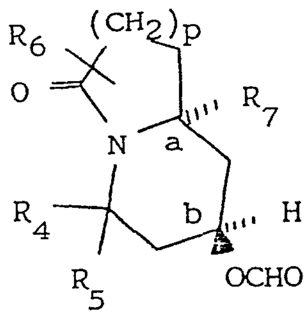

(XVII)

by process which depends on the nature of $R_6$.

When $R_6$ is hydrogen or is other than hydrogen and substitutes the carbon other than the carbon adjacent to the nitrogen, the reaction may be carried out by reduction with, for example lithium aluminium hydride.

When $R_6$ is other than hydrogen and is at the carbon adjacent to the nitrogen, the compound of formula (XVII) wherein $R_6$ is hydrogen is converted firstly with base, such as sodium hydroxide and secondly with a silylating agent, such as $(CH_3)_3SiCl$ or hexamethyldisilazane, to the silyl ether of formula (XVIII):

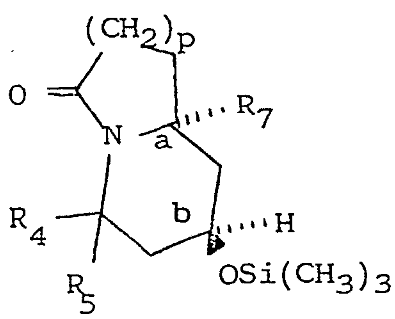

(XVIII)

which in turn is converted to the desired compound of formula(XIV) with a $R_6$ metal complex, such as $R_6Li$, and subsequent reduction, for example with sodium borohydride.

The intermediates of formula (XVII) may themselves be prepared by treating a compound of formula (XIX):

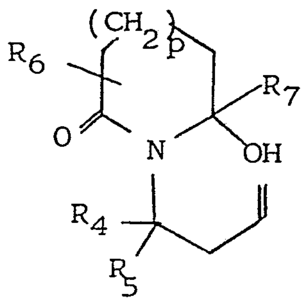

(XIX)

with formic acid.

Compounds of formula (XIX) may be prepared from a compound of formula (XX):

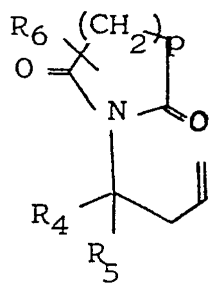

(XX)

by i) when $R_7$ is hydrogen; reduction, for example with sodium borohydride and hydrogen chloride or

ii) when $R_7$ is other than hydrogen; with an $R_7$ organometallic reagent such as $R_7Li$ or $R_7MgX$ wherein X is halogen, which compounds of formula (XX) may themselves be prepared either by reaction of a compound of formula (XXI):

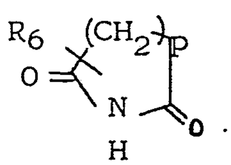

(XXI)

with an alkylene halide, such as 4-brombut-1-ene, using base; or by pyrolyis of a salt of formula (XXII):

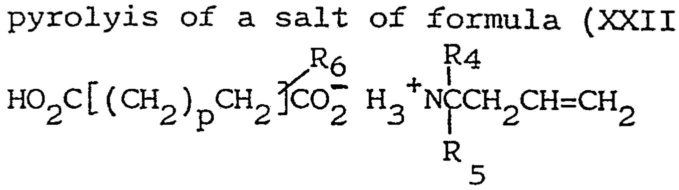

(XXII)

which may be prepared from the corresponding dicarboxcylic acid and primary amine.

Such compounds of the formula (XXII) a may be prepared by the (XXI) - (XX) to (XIX) - (XVII) route. (It will of course be appreciated that in such a reaction sequence the (XX) to (XIX) reduction will give a mixture of isomers which can be separated by conventional means).

Alternatively, when m and n are both 1, the ketone (XV) may be prepared by the acid catalysed condensation of an amine of formula (XXIII):

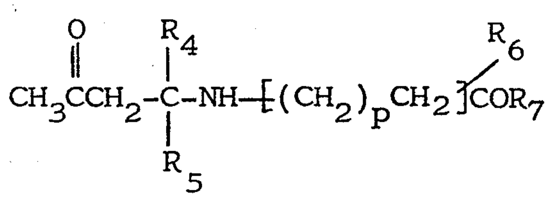 (XXIII)

or a ketal derivative thereof.

The ketone (XV) may also be prepared by cyclisation of a compound of formula (XXIV):

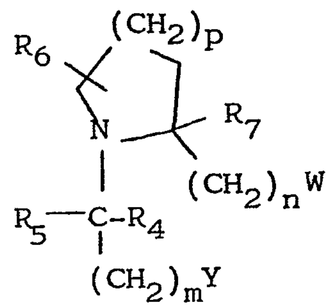

(XXIV)

wherein W and Y are either carboxylic acid ester residues of up to 12 carbon atoms or nitrile.

Suitable values for W and Y when carboxylic acid esters include $C_{1-4}$ alkyl esters such as ethyl esters.

Conditions for this reaction are those normally used for a standard Dieckman cyclisation reaction.

Compounds of formula (XXIII) are either known or may be prepared by standard methods such as (when one of $R_4$, $R_4^1$ is hydrogen) by a conventional addition reaction of between (XXV) and (XXVI):

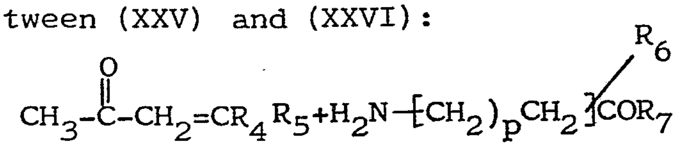

(XXV) (XXVI)

The aforesaid processes of course yield a compound of the formula (XII) wherein $R_8$ is hydrogen.

If a compound of formula (XII)wherein $R_8$ is alkyl is desired, it may simply be prepared from the corresponding $R_8$ is hydrogen compound by alkylation. This may be effected in any suitable manner, for example by acylation with an anhydride followed by reduction with lithium aluminium hydride.

It will be appreciated that any enantiomer, or mixture of enantiomers, of the compounds of formulae (XIII), (XIV), (XVII) & (XVIII) may be used provided it has the depicted spatial relationship between carbons a and b.

The intermediates of the formula (XI) are known compounds, or may be prepared in analogous manner.

The intermediates of formula (XII), (XIII) and (XVI) are novel and thus form an aspect of the present invention.

The N-oxides of the compounds of formula (I) may be prepared in conventional manner, for example by treatment with a per-acid, such as m-chloroperbenzoic acid.

The acid addition salts of compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_6Y$ as defined. This reaction is suitably carried out in an appropriate solvent such as acetone, methanol, ethanol, dimethylformamide, at ambient or raised temperature and pressure.

The skilled man will appreciate that the choice or necessity of conversion of groups $R_2$, $R_3$ and/or $R_{11}$ to other groups $R_2$, $R_3$ and/or $R_{11}$ will be dictated by the nature and position of substituents $R_1$, $R_2$ $R_3$ and $R_{11}$.

It will be apparent that compounds of the formula (I) containing an $R_2$, $R_3$ or $R_{11}$ group which is convertible to another $R_2$, $R_3$ $R_{11}$ group are useful novel intermediates. A number of such conversions is possible not only for the end compounds of formula (I), but also for their intermediates as follows:

(a) an hydrogen substituent is convertible to a nitro substituent by nitration;
(b) a nitro substituent is convertible to an amino substituent by reduction;
(c) a $C_{1-4}$ acylamino substituent is convertible to an amino substituent by deacylation;
(d) an amino substituent is convertible to a $C_{1-4}$ acylamino substituent by acylation;
(e) a hydrogen substituent is convertible to a halogen substituent by halogenation; and
(f) a $C_{1-6}$ alkylthio or $C_{1-6}$ alkylsulphinyl substituent is convertible to a $C_{1-6}$ alkylsulphinyl or a $C_{1-6}$alkyl sulphonyl substituent respectively by oxidation.

Conversions (a) to (f), are only exemplary and are not exhaustive of the possibilities.

In regard to (a), nitration is carried out in accordance with known procedures.

In regard to (b), the reduction is carried out with a reagent suitable for reducing nitroanisole to aminoanisole.

In regard to (c), deacylation is carried out by treatment with a base, such as an alkali metal hydroxide.

In regard to (d) the acylation is carried out with an acylating agent, such as the corresponding acid or acid chloride. Formylation is carried out with the free acid.

In regard to (e), halogenation is carried out with conventional halogenating agents.

In regard to (f), oxidation is carried out at below ambient temperatures in a non-aqueous solvent, such as a chlorinated hydrocarbon, in the presence of an organic peracid, such as 3-chloroperbenzoic acid, or in water in the presence of a soluble strong inorganic oxidant, such as an alkali metal permanganate or in aqueous hydrogen peroxide.

As hereinbefore stated, the compounds of the formula (I) are dopamine antagonists.

The compounds of the formula (I) may be used in the treatment of disorders related to impaired gastro-intestinal motility, such as retarded gastric emptying, dyspepsia, flatulence, oesophagal reflux, peptic ulcer and emesis.

The invention therefore also provides a pharmaceutical composition comprising a compound of the formula (I), or a pharmaceutically acceptable salt thereof, together with a pharmaceutically acceptable carrier.

Such compositions may be adapted for oral or parenteral administration, and as such may be in the form of tablets, capsules, oral liquid preparations,

powders, granules, lozenges, reconsitutable powders, injectable and infusable solutions or suspensions and the like; the compositions may also be in the form of suppositories and the like. Normally, orally administrable compositions are preferred.

Tablets and capsules for oral administration may be in unit dose presentation form, and may contain conventional excipients such as binding agents, fillers, tabletting lubricants, disintegrants, and acceptable wetting agents and the like. The tablets may be coated according to methods well known in normal pharmaceutical practice. Oral liquid preparations may be in the form of, for example, aqueous or oily suspensions, solutions, emulsions, syrups, or elixirs, or may be presented in a dry product for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents, and the like.

For parenteral administration, fluid unit dosage forms are prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to

facilitate uniform distribution of the compound.

As is common practice, the compositions will usually be accompanied by written or printed directions for use in the medical treatment concerned.

It will of course be realised that the precise dosage used in the treatment of any of the hereinbefore described disorders will depend on the actual compound such as the seriousness of the disorder being treated.

The invention further provides a method of treatment of disorders related to impaired gastro-intestinal motility, such as retarded gastric emptying, dyspepsia, flatulence, oesophagal reflux, peptic ulcer or emesis, in mammals, including humans, comprising the administration of a therapeutically effective amount of a compound of the formula (I) or a pharmaceutically acceptable salt thereof.

The "effective amount" will depend in the usual way on a number of factors such as the nature and severity of the malady to be treated, the weight of the sufferer, and the actual compound used.

However, by way of illustration, unit doses will contain 0.005 to 20 mgs, of a compound of formula (I).

Again by way of illustration, such unit doses will suitably be administered more than once per day, for example 2, 3, 4, 5, or 6 times a day, in such a way that the total daily dose is suitably in the range 0.01 to 20 mg/kg per day.

Compounds of the formula (I) have the ability to potentiate the effect of conventional analgesics in migraine treatment when administered concurrently with the analgesic.

Thus the invention provides a pharmaceutical composition comprising a compound of the formula (I) and an analgesic, together with a pharmaceutically acceptable carrier.

The compound of the formula (I) and the analgesic such as aspirin or paracetamol, will be present in the composition in amounts generally similar to their usual effective dose.

The composition can be a combination produce, for example a tablet or capsule containing both a compound of the formula (I) and an analgesic for oral administration, or a twin pack comprising the two active ingredients separately.

The invention also provides a method of treatment of migraine comprising the administration to the sufferer of a therapeutically effective amount of a compound of formula (I) or a pharmaceutically acceptable salt thereof.

The following Examples illustrate the preparation of the compounds of formula (I) and the following Descriptions illustrate the preparation of intermediates

In The Examples, (±) of course indicates that the compound is a racemate. However, for the sake of convenience, the structural formula of only one enantiomer has been shown.

Description 1

(a) (±) 9α,β-Methyl-1-aza-5α-H-bicyclo[4,3,0]nonane-7-one (D.1a) intermediate for compounds 6, 7, 8, 9, 10, 11, 12 and 13

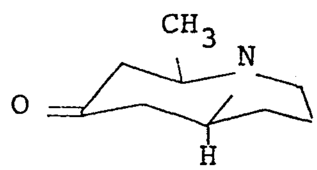

D.1a

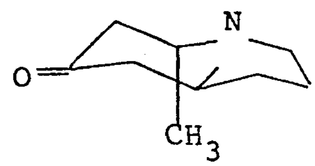

D.1a'

A mixture of 4-aminobutyraldehyde diethyl acetal (3.2 g) and 3-penten-2-one (2.5 g) was allowed to stand at room temperature for 1 hour. Ether (50 ml) and 2N hydrochloric acid (50 ml) were then added and the aqueous layer was separated and heated in a steam bath for 2 hours. The cooled reaction mixture was then concentrated by rotary evaporation and the residue was treated with methylene chloride (100 ml) and saturated potassium carbonate solution. The organic layer was extracted twice more with methylene chloride (50 ml). The combined extracts were dried ($K_2CO_3$) and concentrated and the product was chromatographed (Silica, elution with ethyl acetate) to afford the (±) 9β-methyl-1-aza-5α-H-bicyclo[4,3,0]nonane-7-one (D.1a) (2.0 g, 60%) .

Picrate mp 179$^{o}$ (dec) (ethanol/acetone).

Further elution with ethyl acetate/2% methanol afforded the (±) 9α-methyl-1-aza-5α-H-bicyclo[4,3,0]nonane -7-one (D. 1a')(0.5 g, 15%).
Picrate mp 190-1° (dec) (ethanol/acetone).

Following the procedures outlined above, the following ketones (b) to (d) were prepared:

(b) (±) 9,9-Dimethyl-1-aza-5α-H-bicyclo[4,3,0]nonane -7-one (D.1b) (57%, bp 65° at 1 mm) (picrate mp 179° (dec)), intermediate for compounds 14, 15, 16 and 17

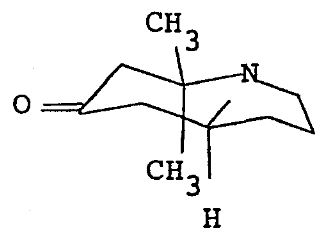

D.1b

(c) (±) 9-Methyl-3-phenyl-1-aza-5α-H-bicyclo[4,3,0]nonan-7-one (D.1c) intermediate for compounds 18, 19, 20, 21, 22 and 23

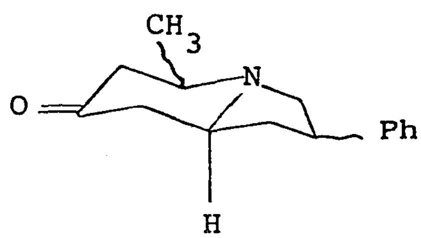

D.1c and D.1c'

Isomer 1 (D.1c) 19%

Isomer 2 + 3 (D.1c') 27%

(d) (±) 2-methyl-1-aza-6α-H-bicyclo[4,4,0]decan-4-one (D.1d) intermediate for compounds 24, 25, 26, 27, 28 and 29

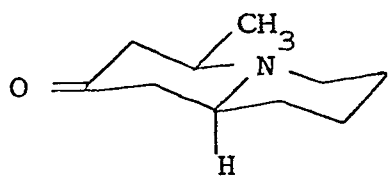

D.1d

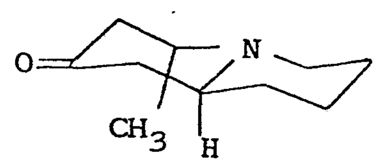

D.1d'

2-β-methyl (D.1d) 57% picrate mp 179-80°
2-α-methyl (D.1d') 21% picrate mp 187-9°

(e) (±) 5α-methyl-1-aza-bicyclo[4,3,0]nonan-7-one (D.1e) intermediate for compounds 30 and 31

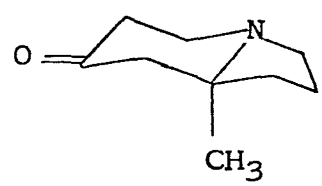

D.1e

To a stirred solution of (±) 7β-hydroxy-5α-methyl-1-azabicyclo[4,3,0]nonane (4 g) and fluorenone (24 g) in dry benzene (100 ml) was added potassium tert-butoxide (8 g) and the solution stirred at ambient temperatures for 30 mins. The product was extracted into 5N hydrochloric acid (50 mls) and washed with ethyl acetate (2 x 100 ml). The aqueous phase was neutralised, then saturated with potassium carbonate and extracted with methylene chloride (3 x 100 ml). Removal of the solvent gave the crude (±) 5α-methyl-1-azabicyclo[4,3,0]nonan-7-one (D.1e) (4.0 g, ca. 100%).

(f) (±) 9α-Ethyl-1-aza-5α-H-bicyclo[4,3,0]nonane-7-one (D.1f) (15%) intermediate for compounds 32 and 33

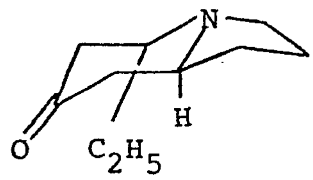

D.1f

(g) (±) 9α-isoPropyl-aza-5α-H-bicyclo[4,3,0]nonane-7-one (D.1g)

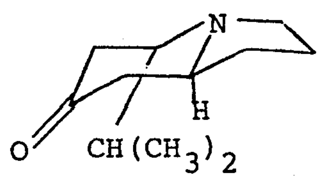

D.1g

Description 2

(a) (±) 2β-Phenyl-1-aza-6α-H-bicyclo[4,4,0]decane-4-one oxime (D.2a) intermediate for compounds 1, 2 and 3

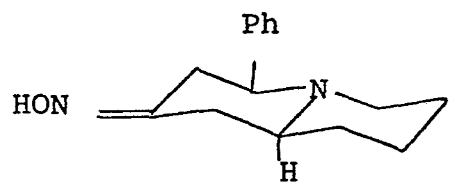

D.2a

An ethanolic (60 ml) solution of (±) 2β-phenyl-1-aza-6α-H-bicyclo[4,4,0]decane-4-one (5.0 g) was treated with pyridine (5 ml) and hydroxylamine hydrochloride (5 g) and the mixture was heated under reflux for 1 hour.

On cooling, the ethanol was removed by rotary evaporation and the residue was treated with dilute potassium carbonate solution. The product was extracted with methylene chloride and dried ($K_2CO_3$). Removal of the solvent gave the crude (±) 2β-phenyl-1-aza-6α-H-bicyclo[4,4,0]decane-4-one oxime (D.2a) (4.8 g, 90%).

Following the procedures outlined above, the following oximes were prepared from the appropriate hydroxylamines:

(b) (±) 2α-Phenyl-1-aza-6α-H-bicyclo[4,4,0]decane-4-one oxime (D.2b) (95%), intermediate for compounds 4 and 5

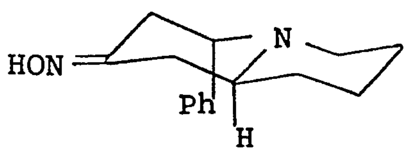

D.2b

(c) (±) 9β-Methyl-1-aza-5α-H-bicyclo[4,3,0]nonane-7-one oxime (D.2c)(80%), intermediate for compounds 6, 7, 8 and 9

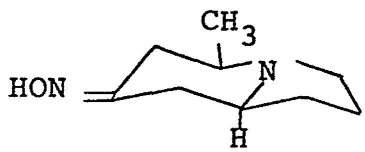

D.2c

(d) (±) 9α-Methyl-1-aza-5α-H-bicyclo[4,3,0]nonane-7-oxime (D.2d) (80%) intermediate for compounds 10 and 11

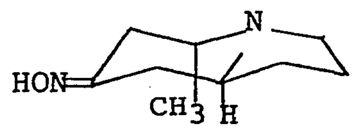

D.2d

(e) (±) 9α-Methyl-1-aza-5α-H-bicyclo[4,3,0]nonane-7-one O-methyl oxime (D. 2e) (100%), intermediate for compounds 12 and 13

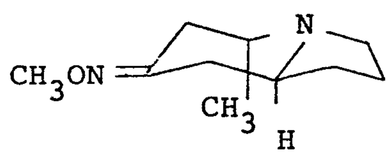

D.2e

(f) (±) 9,9-Dimethyl-1-aza-5α-H-bicyclo[4,3,0]nonane -7-one oxime (D.2f) (90%), intermediate for compounds 14 and 15

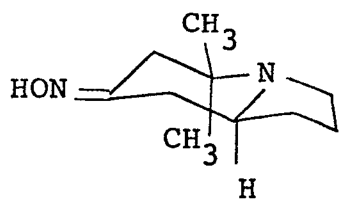

D.2f

(g) (±) 9,9-Dimethyl-1-aza-5α-H-bicyclo[4,3,0]nonane-7-one O-methyl oxime (D.2g) (ca 100%), intermediate for compounds 16 and 17

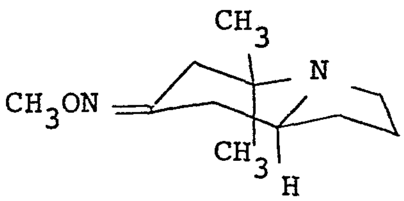

D.2g

(h) (±) 9-Methyl-3-phenyl-1-aza-5α-H-bicyclo[4,3,0]-nonane-7-one oxime, Isomer 1 (D.2h) (ca. 100%), intermediate for compounds 18 and 19

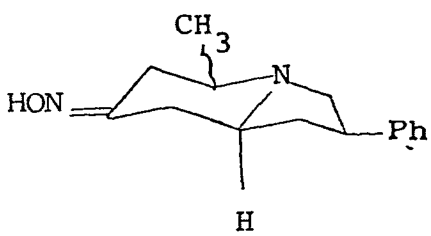

D.2h

(i) (±) 9-Methyl-3-phenyl-1-aza-5α-H-bicyclo[4,3,0]nonane-7-one oxime, Isomer 2 + 3 (D.2i) (ca. 100%), intermediate for compounds 20, 21, 22 and 23

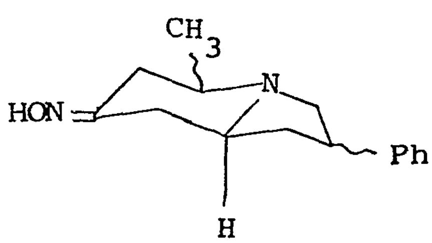

D.2i

(j) (±) 2β-methyl-1-aza-6α-H-bicyclo[4,4,0]decane-4-one O-methyl oxime (D.2j) ca. 100%), intermediate for compounds 24, 25, 26 and 27

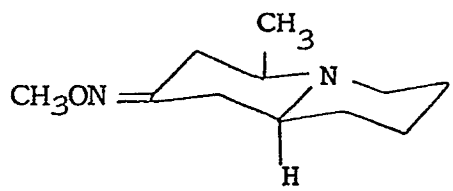

D.2j

(k) (±) 2α-methyl-1-aza-6α-H-bicyclo[4,4,0]decane-4-one O-methyl oxime (D.2h) (ca. 100%), intermediate for compounds 28 and 29

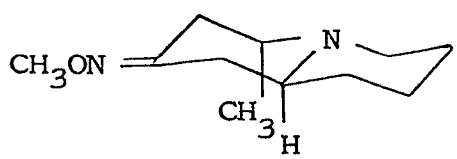

D.2k

(l) (±) 5α-methyl-1-azabicyclo[4,3,0]nonane-7-one oxime (D.21) (ca. 100%), intermediate for compounds 30 and 31

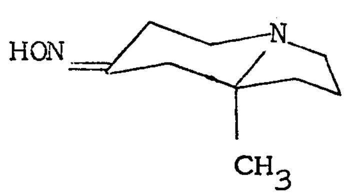

D.21

(m) (±) 9α-Ethyl-1-aza-5α-H-bicyclo[4,3,0]nonane-7-one oxime (D.2m) (in 95%), intermediate for compounds 32 and 33

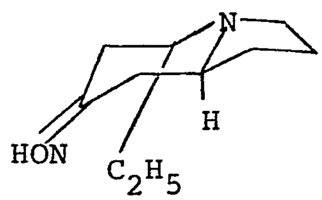

D.2m

m.s. $M^+$ 182.1423 (theory 182.1419)

(n) (±) 9α-isoPropyl-1-aza-5α-H-bicyclo[4,3,0]nonane-7-one oxime (D.2n) (in 100%), intermediate for compounds 34 and 35

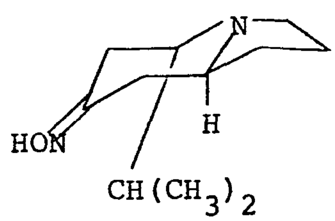

D.2n

m.s. $M^+$ 196.1569 (theory 196.1576)

D.2 (p) (±) 9β-isoPropyl-1-aza-5α-H-bycyclo 4,3,0 nonane-7-one oxime (D.2p) (ca 100%), intermediate for compounds 36 and 37.

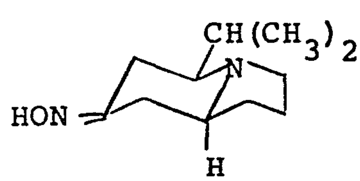

(D.2p)

Description 3

(a) (±) 4α,β-Amino-2β-phenyl-1-aza-6α-H-bicyclo[4,4,0] decane (D.3a) intermediate for Compounds 1, 2 and 3

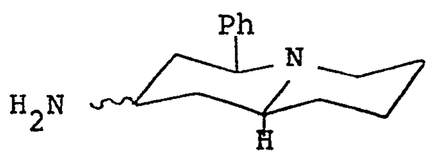

D.3a

To a stirred suspension of lithium aluminium hydride (1.5 g) in dry THF (100 ml) was added, dropwise, a solution of the crude (±) 2β-phenyl-1-aza-6α-H-bicyclo [4,4,0]decane-4-one oxime (D.1) (4.8 g) in dry THF (50 ml) at such a rate as to maintain gentle reflux.

The reaction mixture was boiled under reflux for a further 18 hours, cooled and then the excess hydride was destroyed. Filtration and concentration of the filtrate afforded the crude (±) 4α,β-amino-2β-phenyl-1-aza-6α-H-bicyclo[4,4,0]decane (D.3a) (4.0 g, 85%).

Following the procedures outlined above, the following amines were prepared:

(b) (±) 4α,β-Amino-2α-phenyl-1-aza-6α-H-bicyclo[4,4,0] decane (D.3b) (ca 100%), intermediate for compounds 4 and 5

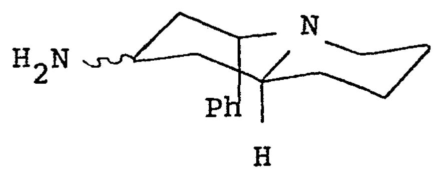

D.3b

(c) (±) 7α,β-Amino-9β-methyl-1-aza-5α-H-bicyclo [4,3,0]nonane (D.3c) (ca 100%), intermediate for compounds 6, 7, 8 and 9

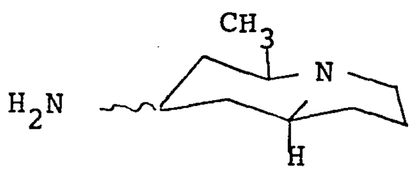

D.3c

D3 (d) (±) 7α,β-Amino-9β-isopropyl-1-aza-5α-H-bicyclo {4,3,0}-nonane (D.3d) (ca 100%), intermediate for compounds 36 and 37

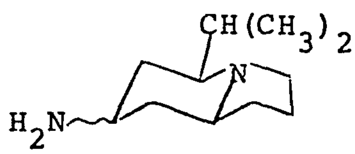

D.3d

Description 4

(a) (±) 7β-Amino-9α-methyl-1-aza-5α-H-bicyclo[4,3,0] nonane (D.4a) intermediate for compounds 10 and 11

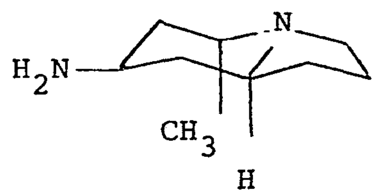

D.4a

A solution of (±) 9α-methyl-1-aza-5α-H-bicyclo [4,3,0]nonane-7-one oxime (D.9) (2.0 g) in amyl alcohol (80 ml) was heated under reflux whilst sodium (ca 2.0 g) was added portionwise over 1 hour. After all the sodium had reacted, the solution was cooled and carefully acidified with 5N hydrochloric acid (ca 60 ml).

Extraction of the amyl alcohol with ethyl acetate (2 x 100 ml), basification and saturation of the aqueous phase with potassium carbonate and re-extraction with methylene chloride (3 x 100 ml) gave the crude (±) 7β-amino-9α-methyl-1-aza-5α-H-bicyclo[4,3,0]nonane (D.4a) (1.5 g, ca 80%).

Following the procedures outlined above, the following amines were prepared:

(b) (±) 7β-Amino-9,9-dimethyl-1-aza-5α-H-bicyclo[4,3,0] nonane (D.4b) (ca 100%), intermediate for compounds 14 and 15

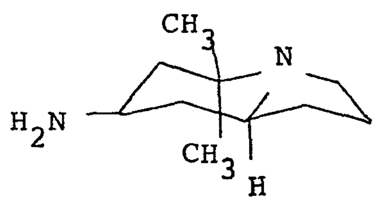

D.4b

(c) (±) 7-β-Amino-9-methyl-3-phenyl-1-aza-5α-H-bicyclo [4,3,0]nonane, Isomer 1 (D.4c) (ca. 100%), intermediate for compounds 18 and 19

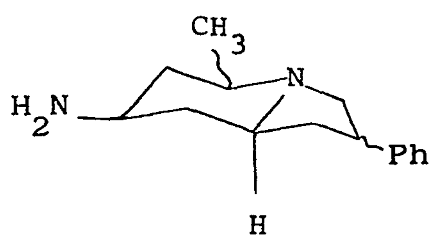

D.4c

(d) (±) 7-β-Amino-9-methyl-3-phenyl-1-aza-5α-H-bicyclo [4,3,0]nonane, Isomer 2 and 3 (D.4d) (ca. 90%), intermediate for compounds 20, 21, 22 and 23

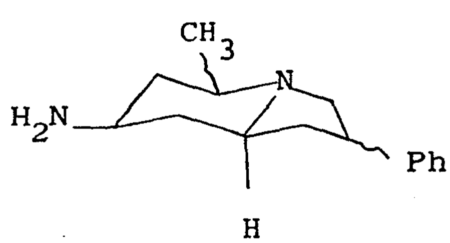

D.4d

(e) $(\pm)$ 7β-Amino-5α-methyl-1-azabicyclo[4,3,0]nonane (D.4e) (50%), intermediate for compound 30 and 31

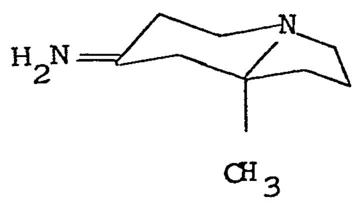

bp 60-63°/1 mm

(f) <u>(±) 7β-Amino-9α-ethyl-1-aza-5α-H-bicyclo[4,3,0]nonane</u> (D.4f) (in 100% crude), intermediate for compounds 32 and 33

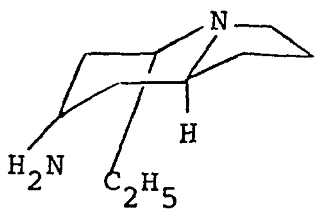

D.4f

(g) <u>(±) 7β-Amino-9α-isopropyl-1-aza-5α-H-bicyclo[4,3,0] nonane</u> (D.4g) (in 100% crude), intermediate for compounds 34 and 35

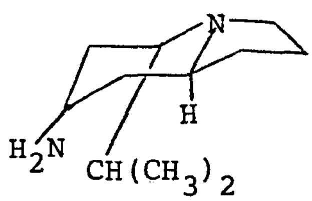

D.4g

Description 5

(a ) (±) 7α-Amino-9α-methyl-1-aza-5α-H-bicyclo[4,3,0] nonane (D.5a) intermediate for compounds 12 and 13

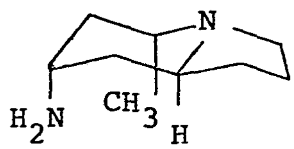

D.5a

A solution of diisobutylaluminium hydride (15 ml of 1.2M) in toluene was added to a stirred solution of (±) 9α-methyl-1-aza-5α-H-bicyclo[4,3,0]nonane-7-one O- oxime (D.10) (1.3 g) in dry THF (100 ml) and the solution was heated under reflux for 18 hours. On cooling, the excess hydride was destroyed, the reaction mixture was filtered. The filtrate was concentrated to give the crude (±) 7α-amino-9α-methyl-1-aza-5α-H-bicyclo[4,3,0]nonane (D.5a) (1.0 g, ca 100%).

Following the procedures outlined above, the following amine was prepared:

(b) (±) 7α-Amino-9,9-dimethyl-1-aza-5α-H-bicyclo [4,3,0]nonane (D.5b) (ca 90%, contains 25% of 7β-isomer), intermediate for compounds 16 and 17

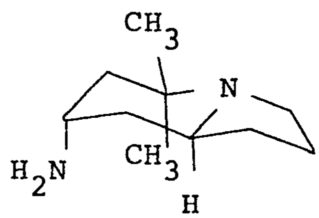

D.5b

(c) (±) 4α,β-Amino-2β-methyl-1-aza-6α-H-bicyclo[4,4,0]-decane (D.5c) (ca. 100%), intermediate for compounds 24, 25, 26 and 27

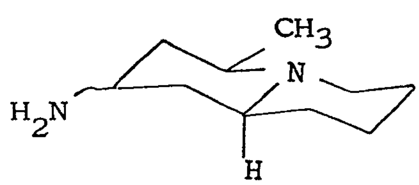

D.5c

(d) (±) 4-β-Amino-2α-methyl-1-aza-6α-H-bicyclo[4,4,0]-decane (D.5d) (ca. 100%), intermediate for compounds 28 and 29

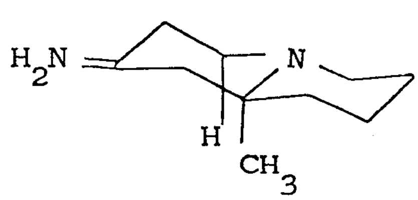

D.5d

Example 1

(±) 4-Acetamido-5-chloro-2-methoxy-N-[4'-(1'-aza-2'β-phenyl-6'α-H-bicyclo[4,4,0]decyl)]benzamide (1a) and (1b)

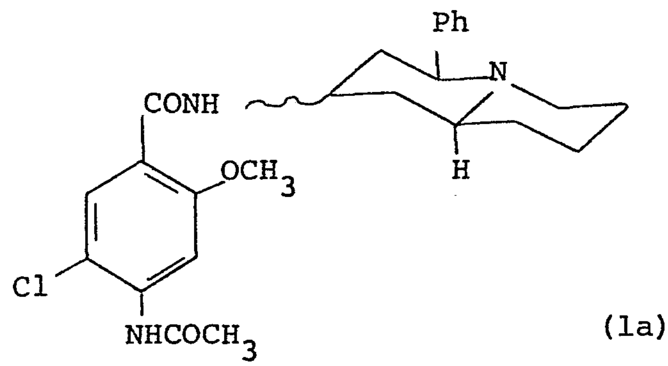

(1a) and (1b)

(α and β forms)

To 4-acetamido-5-chloro-2-methoxybenzoyl chloride (5.7 g) in toluene (250 ml) and triethylamine (6 ml) was added the crude (±) 4α,β-amino-2β-phenyl-1-aza-6α-H-bicyclo[4,4,0]decane (D.3a) (4.0 g) (prepared as in Description 3a) in toluene (20 ml). The reaction mixture was stirred at room temperature for 4 hours, then treated with 2.5N sodium hydroxide solution (20 ml). The toluene layer was separated and the aqueous layer was re-extracted with chloroform (2 x 100 ml) and the combined extracts were dried ($K_2CO_3$). The solvent was removed and the oil was chromatographed (alumina/3% water, ethyl acetate) to yield a mixture of isomers. Trituration with petrol gave one of the isomers of the title compound (1a) (1.0 g, 13%) as a sticky solid.

Concentration of the mother liquors gave an oil containing the other isomer of the title compounds (1b) (3.6 g, 47%).

Example 2

Isomer (A) of (±) 4-amino-5-chloro-2-methoxy-N-[4'-(1'-aza-2'β-phenyl-6α'-H-bicyclo[4,4,0]decyl)]benzamide (2)

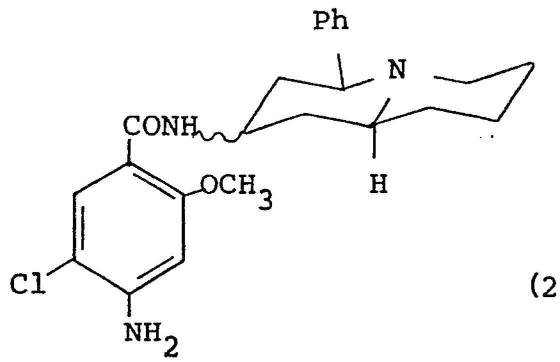

(2)

(1a) (1.0 g) (prepared as in Example 1) was heated under reflux in ethanol (20 ml) and aqueous sodium hydroxide (2.5N, 2 ml) for 2 hours. On cooling, water (100 ml) was added and the solid which formed was collected. Recrystallisation from ethyl acetate/petrol afforded the isomer (A) of (±) 4-amino-5-chloro-2-methoxy-N-[4'-(1'-aza-2'β-phenyl-6'α-H-bicyclo[4,4,0]decyl)]benzamide (2) (0.7 g, 75%), mp 228-30°.

N.B. Isomer (A) is either the axial (α) or equatorial (β) form of the title compound.

Example 3

Isomer (B) of (±) 4-amino-5-chloro-2-methoxy-N-[4'-(1'-aza-2'β-phenyl-6'α-H-bicyclo[4,4,0]decyl)]benzamide (3)

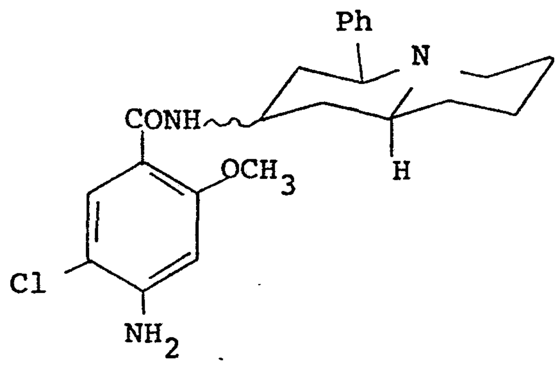

(3)

Following the procedures outlined in Example 2, the crude second isomer of (±) 4-acetamido-5-chloro-2-methoxy-N-[4'-1'-aza-2'β-phenyl-6'β-H-bicyclo[4,4,0] decyl)benzamide (2) (3.6 g) was converted to (±)-4-amino-5-chloro-2-methoxy-N-[4'-(1'-aza-2'β-phenyl-6'α-H-bicyclo [4,4,0]decyl)]benzamide (3) (1.2 g, 35%), mp 204-6°.

N.B. Isomer (B) is either the axial (α) or equatorial (β) form of the title compound, which is not Isomer (A) (Example 2).

Following the procedures outlined in Examples 1 and 2, the following 4-amino and 4-acetamido compounds were prepared. Percentage yields are shown in brackets.

Example 4

(±) 4-Acetamido-5-chloro-2-methoxy-N-[4'α,β-(1'-aza-2'α-phenyl-6'α-H-bicyclo[4,4,0]decyl)]benzamide (4) (26%) as an oil

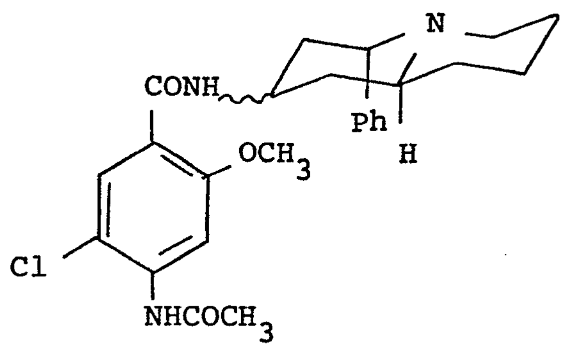

(4)

Example 5

(±) 4-Amino-5-chloro-2-methoxy-N-[4'α,β-(1'-aza-2'α-phenyl-6'α-H-bicyclo[4,4,0]decyl)]benzamide (5)
(80%) mp 233-4$^{O}$ ($CHCl_3$/petrol)

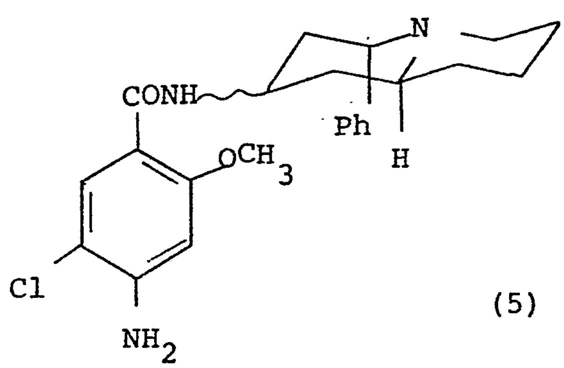

(5)

Example 6

(±) 4-Acetamido-5-chloro-2-methoxy-N-[7'β-(9'β-methyl-1'-aza-5α-H-bicyclo[4,3,0]nonyl)]benzamide (6) (38%) from ethyl acetate trituration of the mixture of α and β isomers

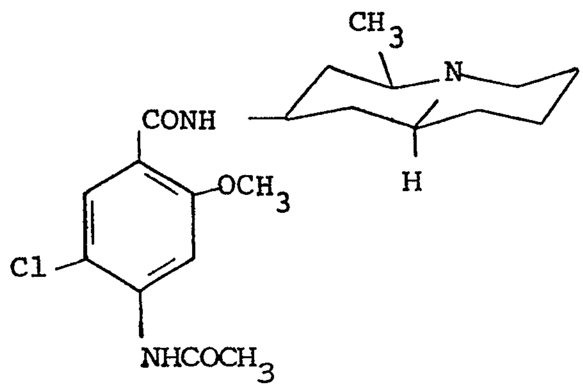

(6)

N.m.r.(δ, $CDCl_3$) : 8.3-7.4 (m, 4H, ArCONH, $CH_3CONH$, including 8.15, s and 8.03, s, both 1H, aromatic H);
4.5-2.9 (m, 6H, aliphatic H, including 3,85, s, 3H, $OCH_3$);
2.7-0.7 (m, 16H, remaining protons including 2.22, s, 3H, $COCH_3$ and 1.1, d, 3H, J=6Hz, $=CHCH_3$).

Example 7

Mainly (±) 4-acetamido-5-chloro-2-methoxy-N-[7'α-(9'β-methyl-1'-aza-5'α-H-bicyclo[4,3,0]nonyl)]benzamide (7) (ca 50%) from concentration of the mother liquors of Example (6)

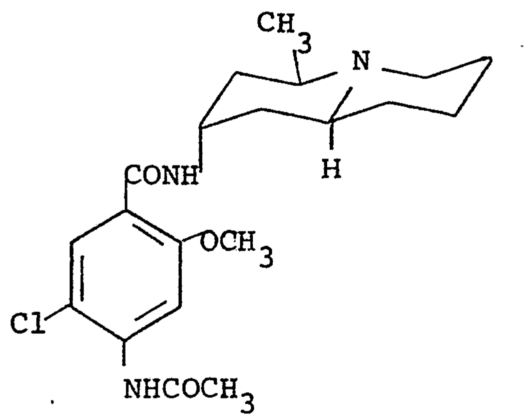

(7)

N.m.r. (δ,$CDCl_3$) : 8.5-7.7 (m, 4H, ArCONH, $CH_3$CONH, including 8.16, s, and 8.04, s, both 1H, aromatic H); 4.7-3.0 (m, 6H, aliphatic H including 3.92, s, 3H, $OCH_3$); 2.7-1.0 (m, 16H, remainign protons including 2.24, s, 3H, $COCH_3$ and 1.12, d, 3H, J=6Hz, $CHCH_3$).

Example 8

(±) 4-Amino-5-chloro-2-methoxy-N-[7'β-(9'β-methyl-1'-aza-5'α-H-bicyclo[4,3,0]nonyl)]benzamide (8) (60%) mp 170-1° (ethyl acetate/petrol) (prepared from Compound 6)

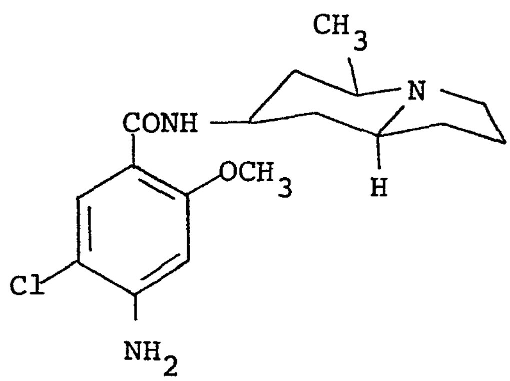

(8)

N.m.r. (δ, $CDCl_3$) : 8.03 (s, 1H, aryl 6H);
7.7-7.3 (m, 1H, CONH);
6.23 (s, 1H, aryl 3H);
4.6-3.6 (m, 6H, $H_2N$, aliphatic H including 3.80, s, 3H, $OCH_3$);
3.45-3.0 (m, 1H, aliphatic H);
2.5-0.9 (m, 14H, remaining protons including 1.11, d, 3H, J=6Hz, $=CHCH_3$).

Example 9

(±) 4-Amino-5-chloro-2-methoxy-N-[7'α-(9'β-methyl-1'-aza-5'α-H-bicyclo[4,3,0]nonyl)]benzamide (9) (40%), mp 238° (ethyl acetate) (prepared from Compound 7)

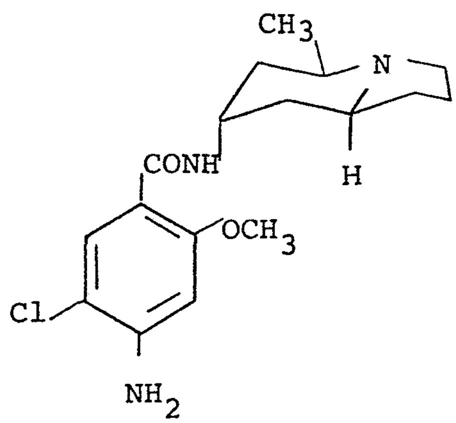

(9)

N.m.r. (δ, $CDCl_3$) : 8.10 (s, 1H, aryl 6H);
8.1-7.9 (m, 1H, CONH);
6.31 (s, 1H, aryl 3H);
4.6-4.3 (m, 3H, $NH_2$ and CONHCH);
3.91 (s, 3H, $OCH_3$);
3.4-3.1 (m, 1H, aliphatic H);
2.5-1.1 (m, 14H, remaining protons including 1.13, d, 3H, J=6.1Hz, $CH_3$).

Example 10

(±) 4-Acetamido-5-chloro-2-methoxy-N-[7'β-(9'α-methyl-1'-aza-5'α-H-bicyclo[4,3,0]nonyl)]benzamide (10) (75%) as an oil

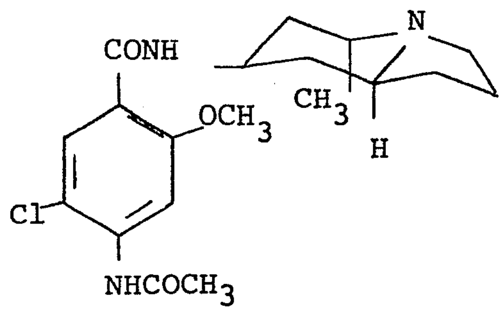

(10)

N.m.r. (δ, $CDCl_3$) : 8.15-7.4 (m, 4H, ArCONH, $CH_3CONH$, including 8.15, s, and 8.05, s, both 1H, aromatic H);
4.5-3.7 (m, 4H, aliphatic H including 3.89, s, 3H, $OCH_3$);
3.6-0.9 (m, 18H, remaining protons including 2.25, s, 3H, $COCH_3$ and 1.05, d, 3H, J=6Hz, $CHCH_3$).

Example 11

(±) 4-Amino-5-chloro-2-methoxy-N-[7'β-(9'α-methyl-1'-aza-5α-H-bicyclo[4,3,0]nonyl)]benzamide (11) (70%) mp 164-6° (ethyl acetate/petrol)

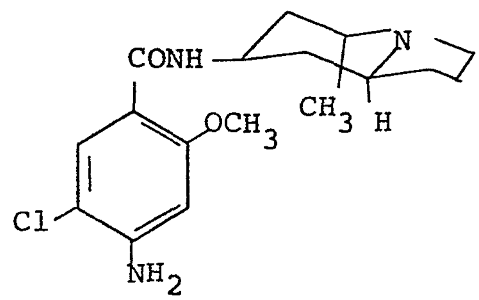

(11)

N.m.r. (δ, $CDCl_3$) : 8.06 (s, 1H, aryl 6H);
7.6-7.4 (m, 1H, CONH);;
6.30 (s, 1H, aryl 3H);
4.65-3.8 (m, 6H, $NH_2$, aliphatic H including 3.82, s, 3H, $OCH_3$);
3.6-3.2 (m, 1H, aliphatic H);
3.0-0.9 (m, 14H, remaining protons including 1.06, d, 3H, J=6.8Hz $CHCH_3$).

Example 12

(±) 4-Acetamido-5-chloro-2-methoxy-N-[7'α-(9'α-methyl-1'-aza-5'α-H-bicyclo[4,3,0]nonyl)]benzamide (12) (70%) as an oil

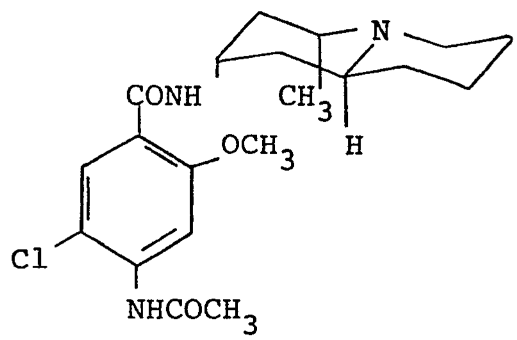

(12)

N.m.r. (δ, $CDCl_3$) : 8.26-7.4 (m, 4H, ArCONH, $CH_3$CONH including 8.26, s, and 8.14, s, both 1H, aromatic H);
4.5-3.4 (m, 5H, aliphatic H including 3.93, s, 3H, $OCH_3$);
3.2-0.8 (m, 17H, remainign protons including 2.26, s, 3H, $COCH_3$ and 0.98, d, 3H, J=7Hz, $CHCH_3$).

Example 13

(±) 4-Amino-5-chloro-2-methoxy-N-[7'α-(9'α-methyl-1'-aza-5α-H-bicyclo[4,3,0]nonyl)]benzamide (13) (40%) mp 169-71° (ethyl acetate/petrol)

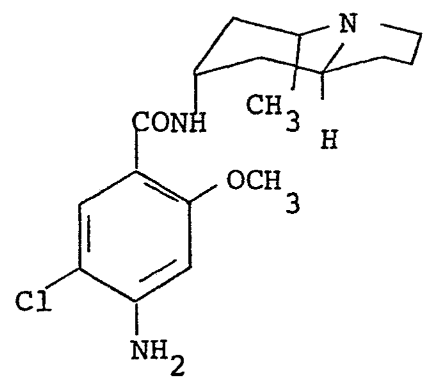

(13)

N.m.r. (δ, $CDCl_3$) : 8.12 (s, 1H, aryl 6H);
7.8-7.5 (m, 1H, CONH);
6.32 (s, 1H, aryl 3H);
4.7-3.8 (m, 6H, aryl $NH_2$, aliphatic H including 3.89, s, 3H, $OCH_3$);
3.8-1.0 (m, 15H, remaining protons including 1.12, d, 3H, J=6Hz, $OCH_3$).

Example 14

(±) 4-Acetamido-5-chloro-2-methoxy-N-[7'β-(9',9'-dimethyl-1'-aza-5'α-H-bicyclo[4,3,0]nonyl)]benzamide (14) (65%) as an oil

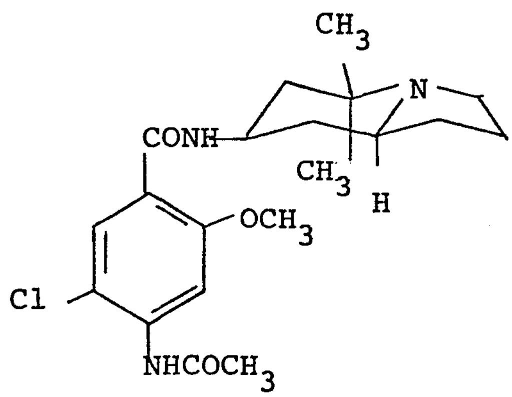

(14)

N.m.r. (δ, $CDCl_3$) : 8.3-7.4 (m, 4H, ArCONH and $CH_3CONH$ including 8.11, s, 1H and 8.01, s, 1H, both aromatic H); 4.6-3.6 (m, 4H, aliphatic H including 3.85, s, 3H, $OCH_3$); 3.1-0.7 (m, 20H, remaining protons including 2.22, s, 3H, $OCH_3$ and 1.17, s, 3H and 1.01, s, 3H both ≡ C-$CH_3$).

Example 15

(±) 4-Amino-5-chloro-2-methoxy-N-[7'β-(9,9'-dimethyl-1'-aza-5'α-H-bicyclo[4,3,0]nonyl)]benzamide (15) (75%) mp 112-5° (ethyl acetate/petrol)

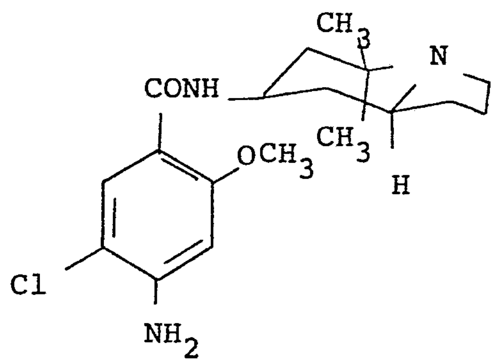

(15)

N.m.r. (δ, $CDCl_3$) : 8.08 (s, 1H, aryl 6H);
7.7-7.3 (m, 1H, CONH);
4.5-3.7 (m, 6H, $NH_2$, aliphatic H including 3.84, s, 3H, $OCH_3$)
3.1-0.6 (m, 18H, remaining protons including 1.18, s, 3H, and 1.04, s, 3H, both $CH_3$).

Example 16

(±) 4-Acetamido-5-chloro-2-methoxy-N-[7'α-(9',9'-dimethyl-1'-aza-5'α-H-bicyclo[4,3,0]nonyl)]benzamide (16) (80%, contains ca 25% of 7'β-isomer) as an oil

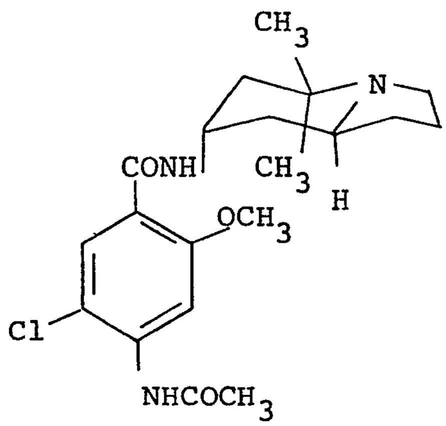

(16)

N.m.r. (δ, $CDCl_3$) : 8.3-7.4 (m, 4H, ArCONH, $CH_3CONH$, including 8.21, s, 1H and 8.13, s, 1H, both aromatic H for 7'α isomer: 8.01, s for 7'β isomer); 4.6-3.6 (m, 4H, aliphatic H including 3.89, s, 3H, $OCH_3$ and shoulder at 3.85 for 7'β isomer); 3.1-0.7 (m, 20H, remaining protons including 2.22, s, 3H, $COCH_3$, and 1.17, 1.12 and 1.01 for $CH_3$ groups).

Example 17

(±) 4-Amino-5-chloro-2-methoxy-N-[7'α-(9',9'-dimethyl-1'-aza-5'α-H-bicyclo[4,3,0]nonyl)]benzamide (17) (60%, contains ca 25% of 7β' isomer) mp 188-9° (ethyl acetate/petrol)

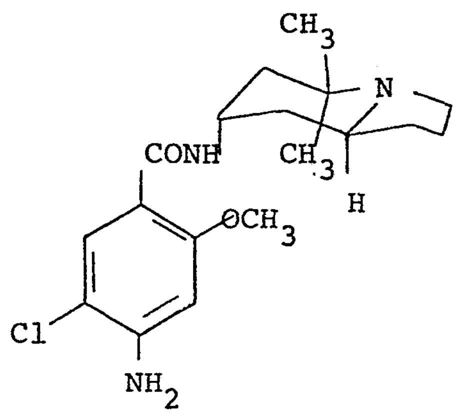

(17)

N.m.r. (δ, $CDCl_3$) : 8.12 (s, 0.75H, aryl 6H);
8.08 (s, 0.25H, aryl 6H) (β isomer);
8.15-7.9 (m, 0.75H, CONH);
7.7-7.3 (m, 0.25H, CONH) (β isomer);
6.30 (6.28) (2 singlets, 1H, aryl 3H);
4.7-4.0 (m, 3H, $NH_2$ and aliphatic H);
3.87 (3.84) (2 singlets, 3H, $OCH_3$);
3.2-0.9 (m, 18H, remaining protons including 1.18, 1.12 and 1.04, singlets for $CH_3$).

Example 18

(±) 4-Acetamido-5-chloro-2-methoxy-N-[7'β-(9'-methyl-3'-phenyl-1'-aza-5'α-H-bicyclo[4,3,0]nonyl)]benzamide (18) (ca. 100%) as an oil

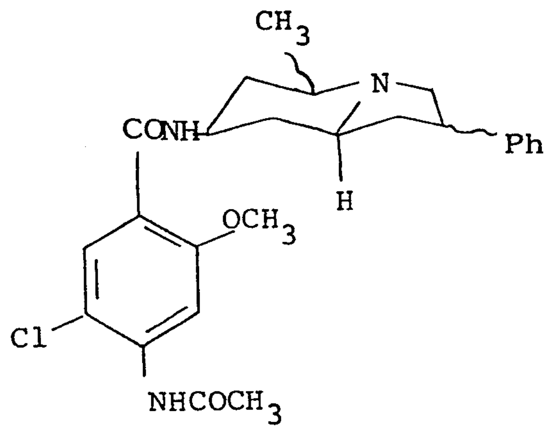

(18)
Isomer 1

n.m.r. (δ, $CDCl_3$) : 8.3-7.4 (m, 4H, ArCONH, $CH_3CONH$, including 8.13, s, 1H and 8.07, s, 1H, both aromatic H);
7.3-6.7 (m, 5H, Aryl H);
4.3-1.0 (m, 21 H, remaining protons including 3.87, s, 3H, $OCH_3$; 2.20, s, 3H, $COCH_3$ and 1.10, d, 3H, $CHCH_3$, J=6$H_z$).

m.s. $M^+$ 455.1967 (Theory 455.1975).

Example 19

(±) 4-Amino-5-chloro-2-methoxy-N-[7'β-(9'-methyl-3'-phenyl-1'-aza-5'α-H-bicyclo[4,3,0]nonyl)]benzamide monohydrochloride (19) (55%) mp 169-71° (ethanol/ether)

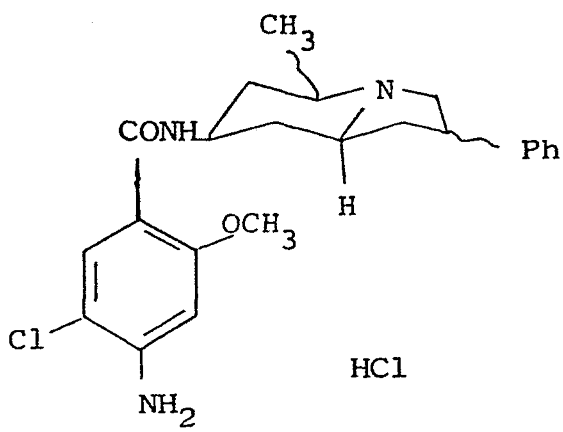

(19)

Isomer 1

n.m.r. (δ, $d^6$DMSO): 8.0-7.1 (m, 7H, CONH and Aryl H);
6.55 (s, 1H, Aryl 3H);
4.4-3.0 (m, 11H, aliphatic protons + $NH_2$ including 3.85, 3H, s, $OCH_3$);
2.6-1.6 (m, 6H, aliphatic protons);
1.35 (d, 3H, $CHCH_3$, J=6$H_z$).

Example 20

(±) 4-Acetamido-5-chloro-2-methoxy-N-[7'β-(9'-methyl-3'-phenyl-1'-aza-5'α-H-bicyclo[4,3,0]nonyl)]benzamide (20) Isomer 2 (56%) as an oil.

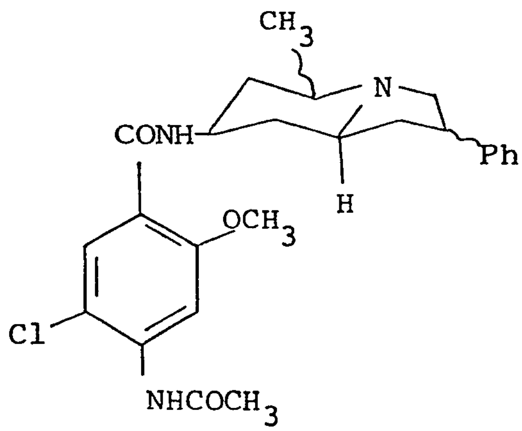

(20)

Isomer 2

Fraction (1) from a silica/ethyl acetate column chromatography.

N.m.r. (δ, $CDCl_3$) : 8.3-7.5 (m, 4H, ArCON$\underline{H}$, $CH_3$CON$\underline{H}$ including 8.17, s, 1H and both aromatic $\underline{H}$)

7.25-6.95 (brs. 5H, Aryl $\underline{H}$);

4.4-0.9 (m, 21H, remaining protons including 3.87, s, 3H, $OC\underline{H}_3$; 2.23, s, 3H, $COC\underline{H}_3$ and 1.13, d, 3H, $CHC\underline{H}_3$, J=6$H_z$).

m.s. $M^+$ 455.1976 (Theory 455.1975).

Example 21

(±) 4-Amino-5-chloro-2-methoxy-N-[7'β-(9'-methyl-3'-phenyl-1'-aza-5'α-H-bicyclo[4,3,0]nonyl)]benzamide (21) Isomer 2 (50%) mp 98-9° (ethyl acetate/petrol)

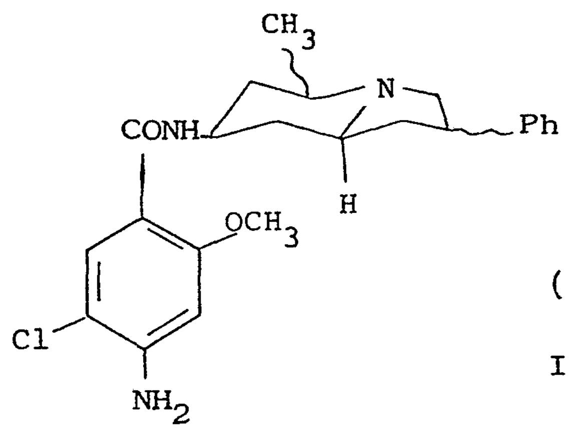

(21)

Isomer 2

n.m.r. (δ, $CDCl_3$) : 8.09 (s, 1H, Aryl 6H);
7.75-7.5 (brd, 1H, CONH);
7.3-7.1 (brs, 5H, aryl H);
6.28 (s, 1H, Aryl 3H);
4.5-3.2 (m, 8H, aliphatic CH and Aryl $NH_2$ including 3.86, s, 3H, $OCH_3$);
2.8-1.8 (m, 7H, aliphatic CH);
1.16 (d, 3H, $CHCH_3$, J=6.2$H_z$).

Example 22

(±) 4-Acetamido-5-chloro-2-methoxy-N-[7'β-(9'-methyl-3'-phenyl-1'-aza-5'α-H-bicyclo[4,3,0]nonyl)]benzamide (22) Isomer 3 (15%) as an oil.

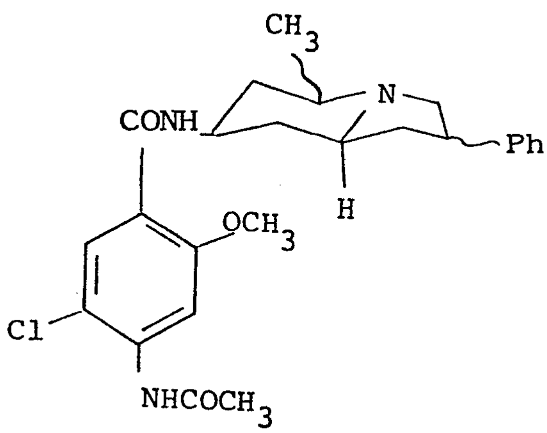

(22)

Isomer 3

Fraction (2) form a silica/ethyl acetate column chromatography.

N.m.r. (δ, $CDCl_3$) : 8.3-6.9 (m, 9H, ArCONH, $CH_3CONH$ including 8.19, s, 1H and 8.10, s, 1H and 7.17, brs, all aromatic H); 4.6-1.0 (m, 21H, remaining protons including 3.86, s, 3H, $OCH_3$; 2.23, s, 3H, $COCH_3$ and 1.12, d, 3H, $CHCH_3$, $J=7H_z$).

m.s. $M^+$ 455.1980 (Theory 455.1975).

Example 23

(±) 4-Amino-5-chloro-2-methoxy-N-[7'β-(9'-methyl-3'-phenyl-1'-aza-5'α-H-bicyclo[4,3,0]nonyl)]benzamide (23) Isomer 3 (55%) mp 229-30° (ethylacetate/petrol)

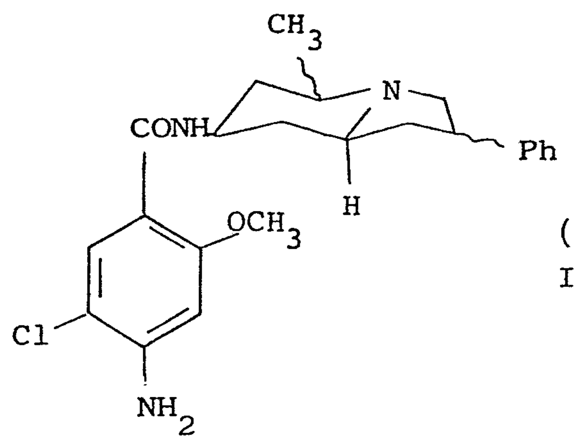

(23)
Isomer 3

N.m.r. (δ, $CDCl_3$) : 8.09(s, 1H, Aryl 6H);
2.65-7.4 (brd, 1H, CONH);
7.25 (brs. 5H, Aryl H);
6.28 (s, 1H, Aryl 3H);
4.6-4.0 (m, 3H, $NH_2$ and NHCH=);
3.86 (s, 3H, $OCH_3$);
3.65-1.0 (m, 14H, remaining protons including 1.13, d, 3H $CHCH_3$, J=6.8$H_z$).

Example 24

(±) 4-Acetamido-5-chloro-2-methoxy-N-[4'β-(7'β-methyl-1'-aza-6'α-H-bicyclo[4,4,0]decyl)]benzamide (24) (70%)

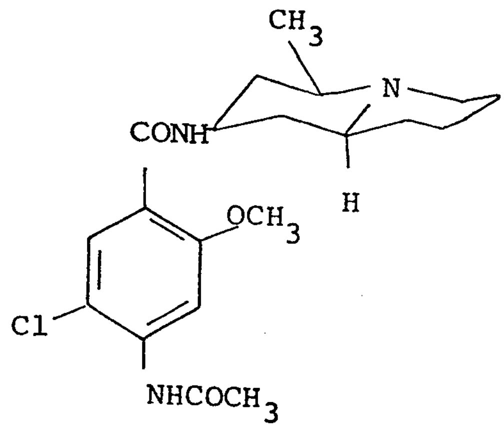

(24)

Isolated from crude reaction mixture (as a sticky solid) by trituration with ether.

N.m.r. (δ, $CDCl_3$) : 8.3-7.5 (m, 4H, ArCONH, $CH_3$CONH including 8.21, s, 1H and 8.10, s, 1H, aryl H);
4.4-1.0 (m, 24H, remaining protons including 3.88, s, 3H, $OCH_3$; 2.23, s, 3H, $COCH_3$ and 1.18, d, 3H, $CHCH_3$, J=6$H_z$).

Example 25

(±) 4-Amino-5-chloro-2-methoxy-N-[4'β-(7'β-methyl-1'-aza-6'α-H-bicyclo[4.4.0]decyl)]benzamide (25) (58%) mp. 184-8° (ethylacetate/petrol)

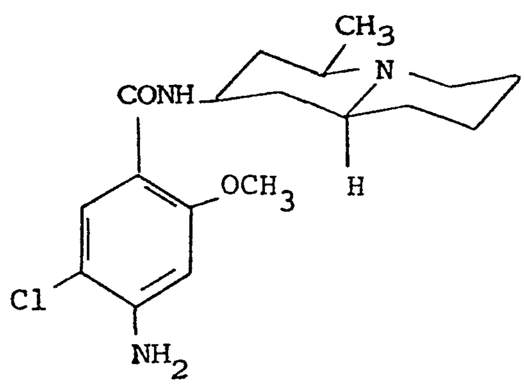

(25)

N.m.r. (δ, $CDCl_3$) : 8.07 (s, 1H, Aryl 6H);
7.7-7.4 (brd, 1H, CONH);
6.29 (s, 1H, Aryl 3H);
4.6-3.75 (m, 6H, Aryl $NH_2$, NHCH = including 3.83, s, 3H, $OCH_3$);
3.4-3.1 (m, 1H, aliphatic H);
2.6-1.0 (m, 16H, remaining protons including 1.12, d, 3H, $CHCH_3$, J=6.1Hz).

m.s. $M^+$ 351.1736 (Theory 351.1759).

Example 26

(±) 4-Acetamido-5-chloro-2-methoxy-N-[4'α-(7'β-methyl-1'-aza-6'α-H-bicyclo[4.4.0]decyl)]benzamide (26) (29%) as an oil containing ca. 10% of 4'β isomer

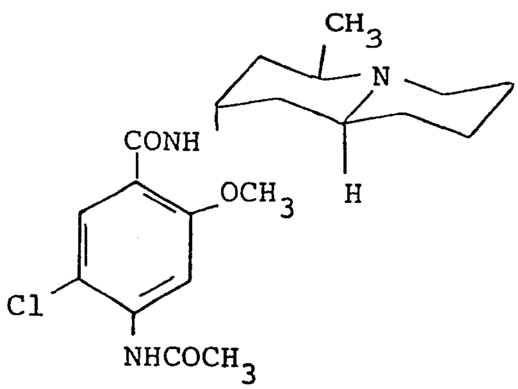

(26)

Isolated from the ether soluble fraction of Example 24.

N.m.r. (δ, $COCl_3$) : 8.3-7.5 (m, 4H, Aryl CON$\underline{H}$, $CH_3$CON$\underline{H}$ including 8.19, s, 1H and 8.09, s, 1H, both aryl $\underline{H}$);
4.6-1.0 (m, 24H, remaining protons including 3.95, s, 3H, $-OC\underline{H}_3$; 2.23, s, 3H, $COC\underline{H}_3$ and 1.12, d, 3H, $CHC\underline{H}_3$, J=6$H_z$).

Example 27

(±) 4-Amino-5-chloro -2-methoxy-N-[4'α-(7'β-methyl-1'-aza-6'α-H-bicyclo[4,4,0]decyl)]benzamide (27) (65%) mp 205-8° (ethylacetate/petrol) contains ca. 10% of 4'β-isomer

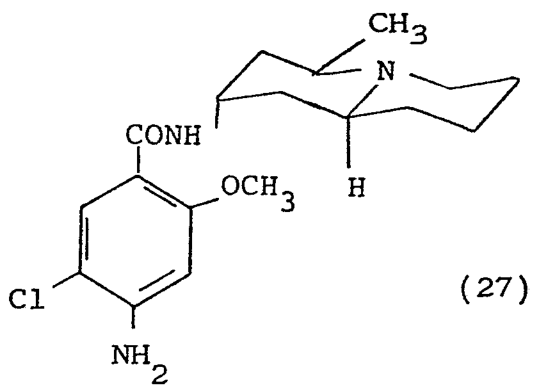

(27)

N.m.r. (δ, $CDCl_3$) : 8.10 (s, 1H, Aryl 6H);
8.25-7.9 (m, 1H, ArCONH);
6.32 (s, 1H, Aryl 3H);
5.6-3.8 (m, 6H, Aryl $NH_2$; NHCH= including 3.94, s, 3H, $OCH_3$);
3.5-3.2 (m, 1H, aliphatic H);
2.5-1.0 (m, 16H, remaining protons including 1.11, d, 3H, $CHCH_3$, J=6Hz).

m.s. $M^+$ 351.1700 (Theory 351.1711).

Example 28

(±) 4-Acetamido-5-chloro-2-methoxy-N-[4'β-(7'α-methyl-1'-aza-6'α-H-bicyclo[4,4,0]decyl)]benzamide (28) (75%) as as oil

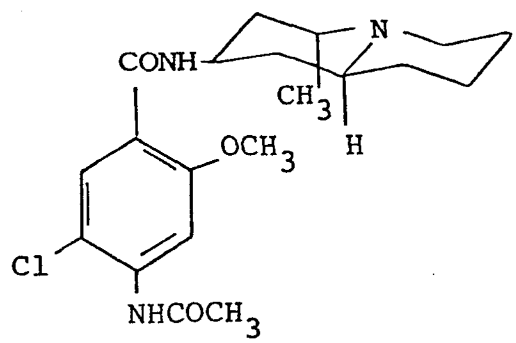

(28)

N.m.r. (δ, $CDCl_3$) : 8.3-7.6 (m, 4H, Aryl CONH, $CH_3$CONH
including 8.19, s, 1H and
8.09, s, 1H, both Aryl H);
4.4-0.9 (m, 24H, remaining protons
including 3.87, s, 3H, $OCH_3$;
2.23, s, 3H, $COCH_3$ and 1.10,
d, 3H, $CHCH_3$, J=7$H_z$).

Example 29

(±) 4-Amino-5-chloro-2-methoxy-N-[4'β-(7'α-methyl-1'-aza-6'α-H-bicyclo[4,4,0]decyl)]benzamide (29) (65%)
mp = 180-3° (ethylacetate/petrol).

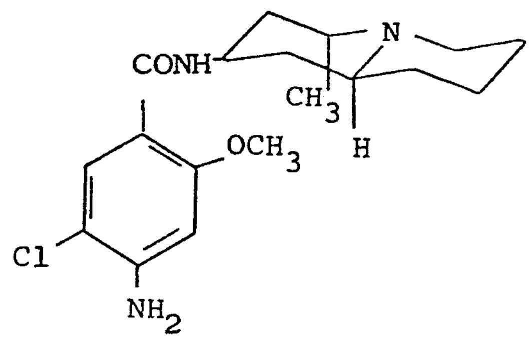

(29)

N.m.r. (δ, $CDCl_3$) : 8.08 (s, 1H, Aryl 6H);
7.8-7.5 (brd, 1H, CONH);
6.31 (s, 1H, Aryl 3H);
4.7-3.7 (m, 6H, Aryl $NH_2$, NHCH= including 3.85, s, 3H, $OCH_3$);
3.4-1.0 (m, 17H, remaining protons including 1.11, d, 3H, $CHCH_3$, J=6.5Hz ).

m.s. $M^+$ 351.1720 (Theory 351.1710).

Example 30

(±) 4-Acetamido-5-chloro-2-methoxy-N-[7'β-(5'α-methyl-1'-aza-bicyclo[4,3,0]nonyl)]benzamide (30) (70%) as an oil

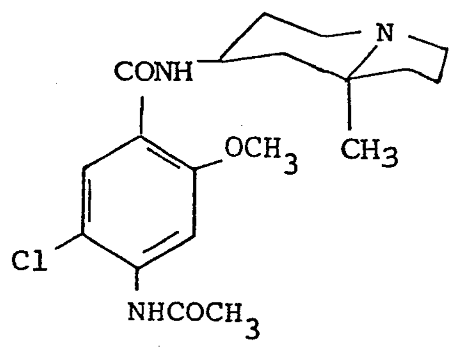

(30)

N.m.r. (δ, $CDCl_3$) : 8.3-7.9 (m, 3H, $CH_3CONH$ including 8.13, s, 1H and 8.03, s, 1H, both aryl H);
7.6-7.3 (brd, 1H, Aryl CONH);
4.4-3.6 (m, 4H, NHCH= including 3.86, s, 3H, $OCH_3$);
3.2-0.9 (m, 18H, remaining protons including 2.25, s, 3H, $COCH_3$ and 1.07, s, ≡C $CH_3$).

Example 31

(±) 4-Amino-5-chloro-2-methoxy-N-[7'β-(5'α-methyl-1'-aza-bicyclo[4,3,0]nonyl)]benzamide (3) (33%) mp 169-71° (ethylacetate/petrol)

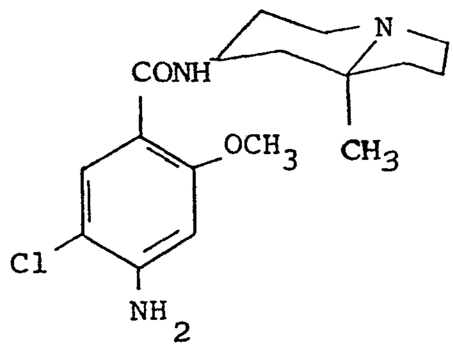

(31)

N.m.r. (δ, $CDCl_3$) : 8.08 (s, 1H, aryl 6H);
7.6-7.3 (brd, 1H, CONH);
6.28 (s, 1H, aryl 3H);
4.6-3.75 (m, 6H, $-NH_2$, NHCH= including 3.86, s, 3H, $-OCH_3$);
3.2-1.0 (m, 15H, remaining protons including 1.08, s, 3H, $CH_3C\equiv$).

Example 32

(±) 4-Acedamido-5-chloro-2-methoxy-N-[7'β-(9'α-ethyl-1'-aza-5' -H-bicyclo[4,3,0]nonyl)]benzamide (32) (90%) mp 108°

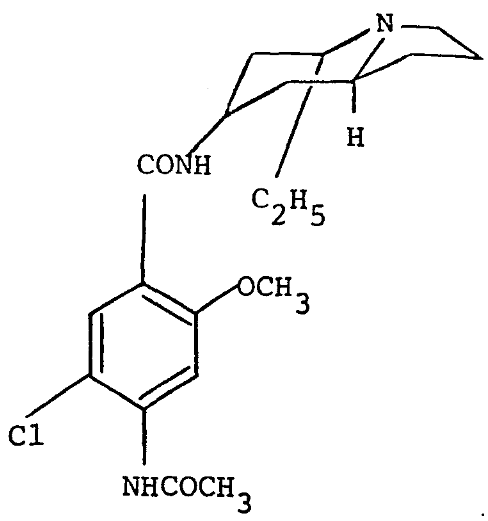

(32)

N.m.r. (δ, $COCl_3$) : 8.3-7.4 (m, 4H, ArCONH, $CH_3$CONH including 8.23, s, and 8.10, s, both 1H, aromatic H); 4.5-0.7 (m, 24H, remaining protons including 3.82, s, 3H, $OCH_3$ and 2.20, s, 3H, $COCH_3$).

m.s. $M^+$ 393.1827 (theory 393.1819)

Example 33

($\pm$) 4-Amino-5-chloro-2-methoxy-N-[7'β-(9'α-ethyl-1'-aza-5'α-H-bicyclo[4,3,0]nonyl)]benzamide (33) (75%) converted to the monohydrochloride mp 119-21$^{o}$ (Ethanol/ether).

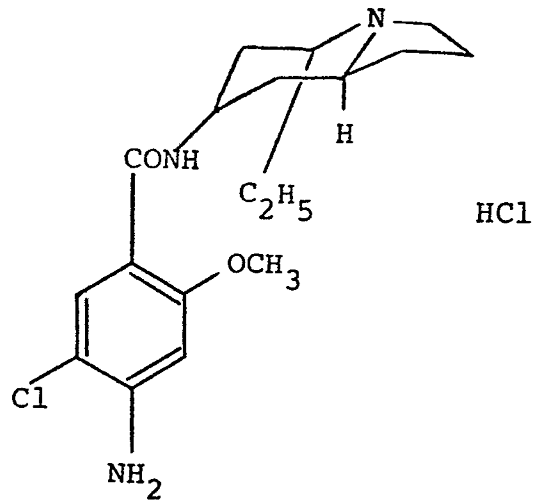

HCl

(33)

N.m.r. (δ, $COCl_3$) of Free Base : 8.09 (s, 1H, aryl 6H);
7.7-7.4 (m, 1H, CONH);
6.27 (s, 1H, aryl 3H);
4.5-3.8 (m, 6H, $NH_2$ aliphatic H including 3.85, s, 3H, $OCH_3$)
3.2-0.8 (m, 17H, remaining protons including 0.93, t, 3H, J=6.9$H_3$, C-$CH_3$).

m.s. $M^+$ 351.1695 (theory 351.1711)

Example 34

(±) 4-Acetamide-5-chloro-2-methoxy-N-[7'β-(9'α-isopropyl-1'-aza-5'-α-H-bicyclo[4,3,0]nonyl)]benzamide (34) (55%) as an oil

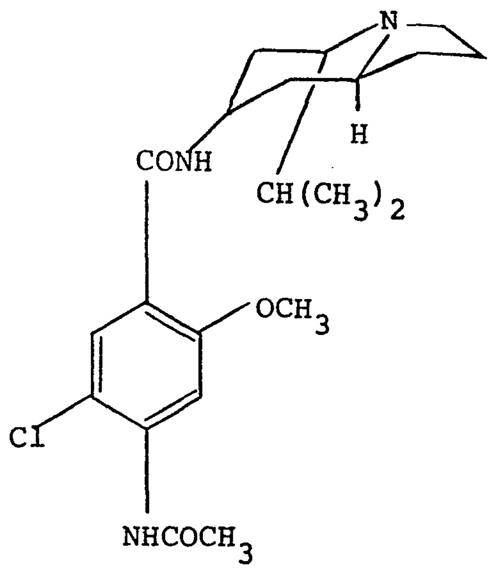

(34)

N.M.F. (δ, $COCl_3$) : 8.3-7.4 (m, 4H, ArCONH-, $CH_3CONH$ including 8.26, s, and 8.16, s, both 1H, aromatic H).

4.5-0.7 (m, 26H, remaining protons including 3.92, s, 3H, $OCH_3$; 2.25, s, 3H, $COCH_3$ and 0.98, d, 6H, $CH(CH_3)_2$).

m.s. M+ 408.2064 (theory 408.2051)

Example 35

(±) 4-Amino-5-chloro-2-methoxy-N-[7'β-(9'α-isopropyl-1'-aza-5'-α-H-bicyclo[4,3,0]nonyl)]benzamide (35) (50%) mp 153-5° (ethylacetate/petrol)

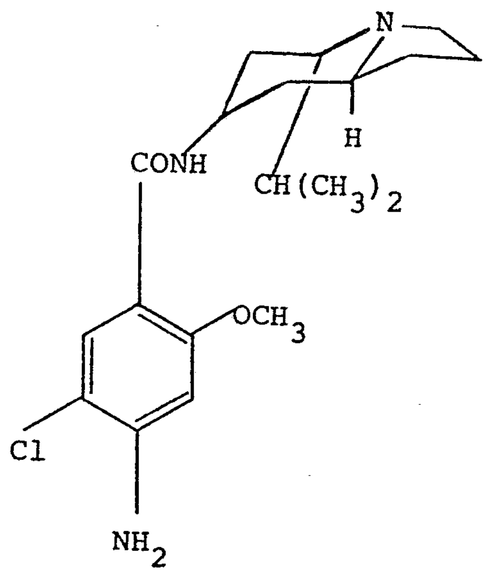

(35)

N.m.r. (δ, $COCl_3$) : 8.10 (s, 1H, aryl 6H);
7.7-7.3 (m, 1H, CONH);
6.28 (s, 1H, aryl 3H);
4.7-3.7 (m, 6H, $NH_2$, aliphatic H including 3.88, s, 3H, $OCH_3$);
3.4-0.7 (m, 19H, remaining protons including 0.97, d, 6H, $J=6.3H_3$; $CH(CH_3)_2$).

m.s. $M^+$ 322.1317 (theory 322.1322)

Example 36

(±) 4-Acetamido-5-chloro-2-methoxy-N-[$7\bar{\alpha}$,β-($9^{-}\beta$-isopropyl-$1^{-}$-aza-$5\bar{\alpha}$-H-bicyclo{4,3,0}nonyl)]benzamide (3b) (65%)

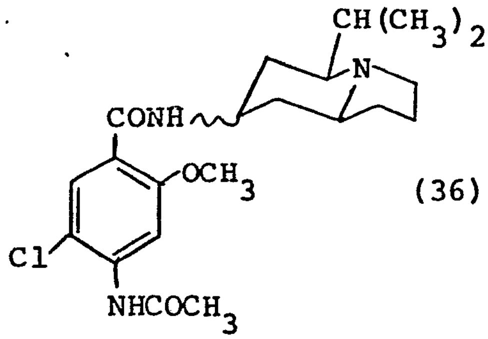

(36)

Trituration of the crude reaction mixture with ether/petrol afforded, as a sticky solid, mainly the (±) 4-acetamido-5-chloro-2-methoxy-N-[$7^{-}\beta$-($9^{-}\beta$-isopropyl-$1^{-}$-aza-$5^{-}\alpha$-H-bicyclo{4,3,0}nonyl)]-benzamide (30%) containing ca. 25% of the $7^{-}\alpha$-isomer.

The mother liquors contained the $7^{-}\alpha$ and $7^{-}\beta$-isomers as approximately a 1:1 mixture (35%).

Example 37

(±) 4-Amino-5-chloro-2-methoxy-N-[7⁻β-(9⁻β-isopropyl-1⁻-aza-5⁻α-H-bicyclo{4,3,0}nonyl)]benzamide (37) (75%) (contains ca. 25% of the 7⁻α-isomer) mp 174-6°

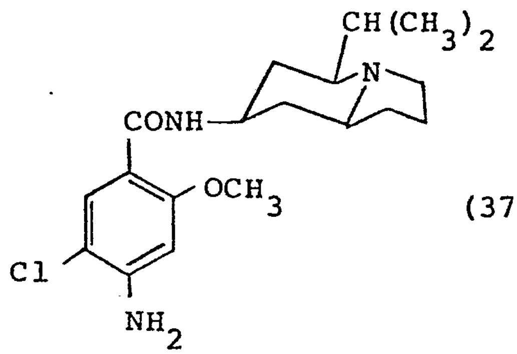

(37)

NMR (δ, $COCl_3$) 8.10 (s, 1H, aryl 6H)

8.1-7.8; 7.7-7.4 (m, 1H, CONH α and β respectively)

6.28 (s, 1H, aryl 3H)

4.7-3.8 (m, 6H, $NH_2$, aliphatic H including 3.90, 3.86, 2s, 3H, $OCH_3$, α and β isomers respectively)

3.3-2.9 (m, 1H, aliphatic H)

2.3-0.7 (m, 18H, remaining protons including 0.88, d, 6H, J=6.5Hz, $CH(CH_3)_2$).

## Pharmacological Data

### Increase in intragastric pressure

Intragastric pressure changes were recorded from previously starved conscious but restrained rats using a saline filled catheter inserted into the lumen of the stomach *via* a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for 40 minute period after administration of compound. Student's "t" test was applied to the difference in average values obtained for spontaneous and post compound activity.

Compounds 8, 9 and 11 significantly increased the index of activity post administration at a dose level of 1.0 mg/kg s.c.

### Anti-emetic activity in the dog

Compounds were administered subcutaneously 30 minutes prior to administration of a standard dose of apomorphine HCl (0.1 mg/kg subcutaneously) and the vomiting response compared to that obtained when the same animals were dosed with apomorphine HCl and vehicle only. The $ED_{50}$ values for inhibition of the vomiting response determined for compound 11 and 13 were 1.0 mg/kg; for compound 33, 0.1 mg/kg and for compound 35, 0.01 mg/kg.

## Dopamine Receptor Blocking Activity in the Central Nervous System

Compounds were tested for inhibition of apomorphine induced climbing in the mouse. The test is based on that described by Protais, P., Constantin, J. and Schwartz J.C. (1976), Psychopharmacology, 50, 1.6.

Apomorphine 1 mg/kg s.c. induces mice to climb the wall of a wire cage (inverted food hopper - 11 x 7.5 x 18 cm high). Mice acclimatised in their home cages in groups of 5 are placed under the hoppers immediately after the injection of apomorphine 1 mg/kg s.c. At 10,20 and 30 minutes after injection climbing behaviour is scored. The mice are observed for 30 seconds and scored according to the position they spend the majortiy of time in, score 0 - four paws on floor of cage; score 1 - four paws only on walls; score 2 - all paws on wall of cage. The scores at all 3 times and for each mouse are summed and mice drug treated orally compared to mice receiving apomorphine only. A saline only treated group is also included and any score, generally>5% of maximum taken into account.

The results were as follows:

| Compound No. | $ED_{50}$ mg/kg s.c. |
|---|---|
| 8 | 10 |
| 9 | 50 |
| 11 | 34 |
| 13 | I 50 |

I = inactive

## Toxicity

No toxic effects were observed in any of the above tests.

Claims

1. A compound of formula (I):

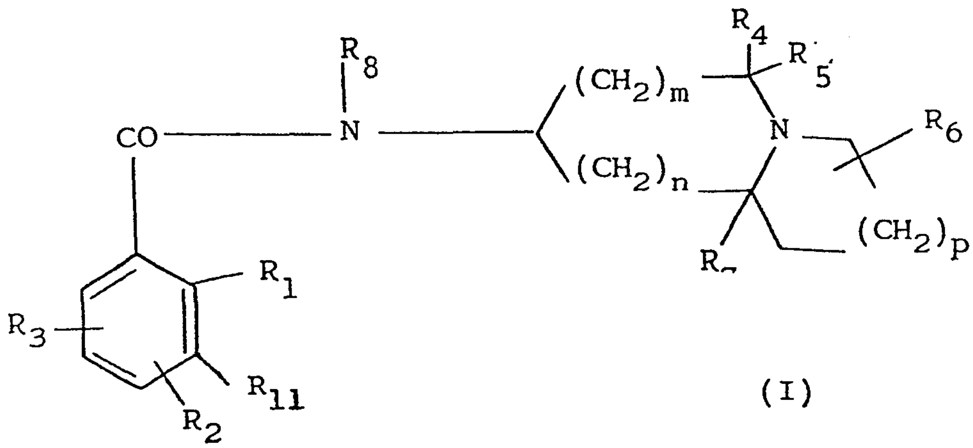

(I)

and pharmaceutically acceptable salts and N-oxides and solvent adducts thereof, wherein:

$R_1$ is $C_{1-6}$ alkoxy, $C_{1-6}$ alkylthio or, together with $R_8$ is $C_{1-2}$ alkylene;

one of $R_2$, $R_3$ and $R_{11}$ is hydrogen and the other two together are $C_{1-2}$ alkylenedioxy, or the other two are the same or different and are selected from the class of hydrogen, halogen, $CF_3$, $C_{1-6}$ alkyl, $C_{1-6}$ alkoxy, $C_{1-6}$ alkythio, $C_{1-7}$ acyl, $C_{1-7}$ acylamino, $C_{1-6}$ alkylsulphonyl, $C_{1-6}$ alkylsulphinyl, hydroxy, nitro or amino, aminocarbonyl or aminosulphonyl optionally N-substituted by one or two $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups or optionally N-disubstituted by $C_{4-5}$ polymethylene; or

$R_1$ and $R_{11}$ together are $C_{1-2}$ alkylenedioxy and $R_2$ and $R_3$ together are $C_{1-2}$ alkylenedioxy or $R_2$ and $R_3$ and the same or different and are selected from the previously defined class of substituents;

$R_4$, $R_5$, $R_6$ and $R_7$ are the same or different and are hydrogen, $C_{1-6}$ alkyl, phenyl or phenyl-$C_{1-4}$ alkyl, either of which phenyl moieties may be substituted by $C_{1-6}$ alkyl, $C_{1-6}$ alkoxy, $CF_3$ or halogen, and at least one of $R_4$, $R_5$ and $R_7$ is other than hydrogen;

$R_8$ is hydrogen or $C_{1-4}$ alkyl.or as defined with $R_1$;

p is 0 to 3;

m is 0 to 3; and

n is 0 to 3.

2. A compound according to claim 1 of formula (VII)

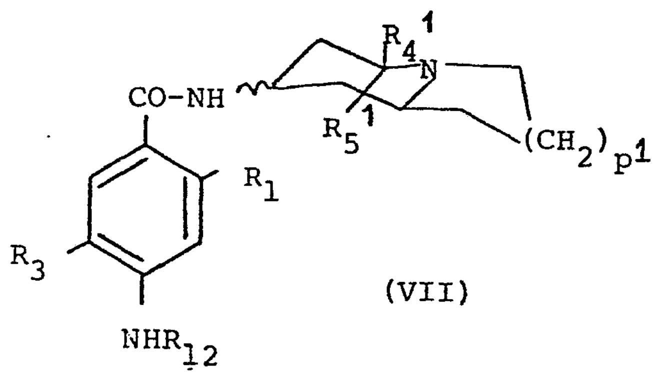

(VII)

wherein $R_1^1$ is $C_{1-6}$ alkoxy;

$R_{12}$ is hydrogen or $C_{1-4}$ alkanoyl;

$R_3^1$ is halogen

$R_4^{\ 1}$ is $C_{1-6}$ alkyl, phenyl or phenyl $C_{1-4}$ alkyl, either of which phenyl moieties may be substituted as defined in claim 1;

$R_5^{\ 1}$ is hydrogen, , $C_{1-6}$ alkyl, phenyl or phenyl $C_{1-4}$ alkyl, either of which phenyl moeities may be substituted as defined in claim 1;

$p^1$ is 1 or 2;

and the bond indicated may be axial or equatorial.

3. A compound according to claim 2 wherein $R_5^{\ 1}$ is hydrogen and $p^1$ is 1.

4. A compound according to claim 1 of formula (X):

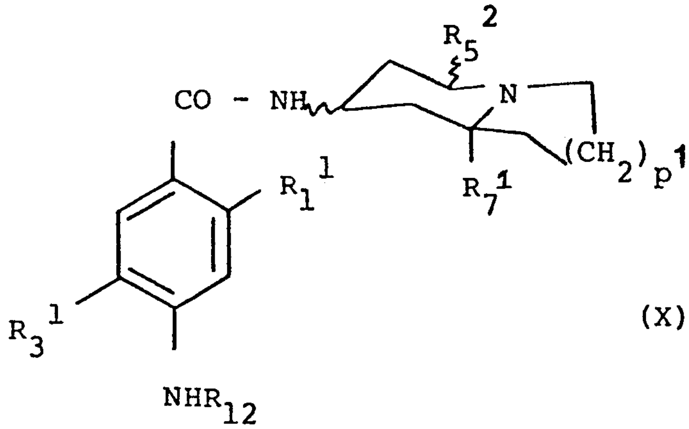

(X)

wherein $R_5^2$ is hydrogen, $C_{1-6}$ alkyl, phenyl or phenyl $C_{1-4}$ alkyl, either of which phenyl moieties may be substituted as defined in claim 1;

$R_7^1$ is $C_{1-6}$ alkyl, phenyl or phenyl $C_{1-4}$ alkyl, either of which phenyl moieties may be substituted as defined in claim 1.

5. A compound according to claim 4 wherein $R_5^2$ is hydrogen.

6. A compound according to any one of the claims 1 to 5 wherein the bond between the benzamide moiety and the cyclic side chain (ie between the $R_8$ substituted nitrogen atom is joined) is equatorial.

7. A compound according to claim 1 which is

(±) 4-Amino-5-chloro-2-methoxy-N-[7'β-(9'β-methyl-1'-aza-5'α-H-bicyclo[4,3,0] nonyl)] benzamide,

(±) 4-Amino-5-chloro-2-methoxy-N-[7'α-(9'β-methyl-1'-aza-5'α-H-bicyclo[4,3,0]nonyl)] benzamide,

(±) 4-Amino-5-chloro-2-methoxy-N-[7'β-(9'α-methyl-1'-aza-5α-H-bicyclo[4,3,0]nonyl)] benzamide, or

(±) 4-Amino-5-chloro-2-methoxy-N-[7'α-methyl -1'-aza-5α-H-bicyclo[4,3,0)] nonyl)]benzamide, or a pharmaceutically acceptable salt thereof.

8. A process for the preparation of a compound according to claim 1, characterised by reacting a compound of formula (XI):

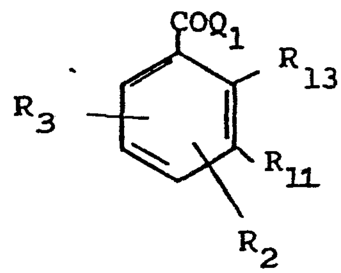

(XI)

with a compound of formula (XII):

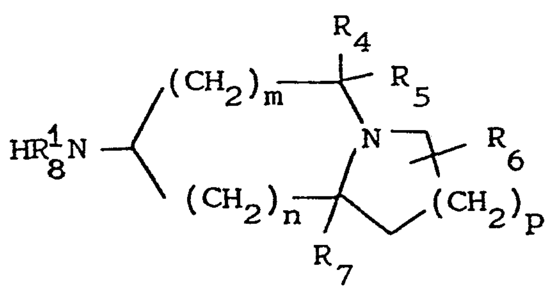

(XII)

wherein $Q_1$ is a leaving group;

$R_{13}$ is $R_1$ as defined or $(CH_2)_qCR_{14}R_{15}$ $Q_2$ wherein q is O or 1, $R_{14}$ and $R_{15}$ are both hydrogen or together form an oxo group and $Q_2$ is a leaving group or $Q_1$ and, $Q_2$ are joined to form a bridging oxygen atom;

$R_8^1$ is hydrogen or $C_{1-4}$ alkyl; and the remaining variable groups are as defined in claim 1; and thereafter when $R_{13}$ is $(CH_2)_qCR_{14}R_{15}$ $Q_2$ and $R_{15}$ form an oxo group, reducing the resulting compound and optionally converting a group $R_2$,$R_3$ or $R_{11}$ in the thus formed compound to another group $R_2$,$R_3$ or $R_{11}$ respectively.

9. A pharmaceutical composition comprising a compound according to any one of the claims 1 to 7, or a pharmaceutically acceptable salt thereof, together with a pharmaceutically acceptable carrier.

10. A compound according to any one of claims 1 to 7 for use in treatment of disorders related to impaired gastric motility.

0067565

European Patent Office

# EUROPEAN SEARCH REPORT

Application number

EP 82 30 2660

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | EP-A-0 013 138 (BEECHAM) *Complete patent* --- | 1,8-10 | C 07 D 471/04<br>C 07 D 455/02<br>A 61 K 31/435//<br>(C 07 D 471/04<br>C 07 D 221/00<br>C 07 D 209/00 ) |
| D,X | DE-A-2 748 260 (BEECHAM) *Complete patent; examples 1-10,15-17* --- | 1,8-10 | |
| P,X | EP-A-0 036 269 (BEECHAM) *Complete patent* ----- | 1,8-10 | |
| | | | TECHNICAL FIELDS SEARCHED (Int. Cl. 3) |
| | | | C 07 D 471/00<br>C 07 D 455/00<br>A 61 K 31/00 |

The present search report has been drawn up for all claims

| Place of search | Date of completion of the search | Examiner |
|---|---|---|
| THE HAGUE | 23-09-1982 | NUYTS A.M.K.A. |

CATEGORY OF CITED DOCUMENTS

X particularly relevant if taken alone
Y particularly relevant if combined with another document of the same category
A technological background
O non-written disclosure
P intermediate document
T theory or principle underlying the invention
E : earlier patent document, but published on, or after the filing date
D document cited in the application
L document cited for other reasons
& member of the same patent family, corresponding document

EPO Form 1503 03 82

BAD ORIGINAL